# EUROPEAN PATENT APPLICATION

(11) **EP 4 541 891 A1**
(43) Date of publication of application: **23.04.2025**
(21) Application number: 23849801.8
(22) Date of filing: 29.06.2023
(51) Int. Cl.: C12N 15/11, A61K 38/10, A61K 38/16, A61K 47/64, A61P 25/18, C07K 7/08, C07K 17/00, C12N 1/19, C12N 1/21, C12N 7/01, C12N 15/63

(54) **NOVEL LRP1-BINDING PEPTIDE**

(30) Priority: 05.08.2022 JP 2022125238
(71) Applicant: Ichimaru Pharcos Co., Ltd., Motosu-shi, Gifu 501-0475 (JP)
(72) Inventor: SAKAMOTO Kotaro, Motosu-shi, Gifu 501-0475 (JP)
(74) Representative: Hasegawa, Kan
(86) International application number: PCT/JP2023/024137
(87) International publication number: WO 2024/029242

(57) **Abstract**

Disclosed is a linear or cyclic peptide having an amino acid sequence represented by the formula (1): X^{N}-X⁵- X⁶-X⁷-X⁸-X⁹-X¹⁰-X¹¹-X¹²-X¹³-X¹⁴-X¹⁵ (1), or a pharmaceutically acceptable salt thereof. The peptide, when bound to LRP1, can deliver any drug to brain tissues by transcytosis mediated by LRP1.

## Description

### CROSS-REFERENCE TO RELATED APPLICATIONS

This application claims priority to Japanese Patent Application No. 2022-125238 filed on August 5, 2022 in Japan, the entire contents of which are incorporated herein by reference.

### TECHNICAL FIELD

The present invention relates to a novel LRP1-binding peptide. The present invention relates to a peptide capable of passing through barrier tissues, for example, the blood-brain barrier (BBB) separating peripheral tissues and brain tissues, when bound to LRP1. More particularly, the present invention relates to a linear or cyclic peptide having a specific amino acid sequence.

### BACKGROUND ART

Central nervous system diseases (mental disorder) such as dementia, schizophrenia, and autism spectrum disorder are estimated to affect as many as one billion people worldwide, and account for the largest number of disability-adjusted life years, thereby casting enormous social burdens. For example, the total economic burden in the United States and Europe in 2030 is estimated at six trillion dollars (Non-Patent Document 1). Against this background, successful drug discovery for the central nervous system diseases is expected to lead to a huge market, but such discovery is also known to have the highest failure rate (Non-Patent Document 1). One of the causes is the presence of barrier tissues separating peripheral tissues and brain tissues in a living body: the blood-brain barrier, the blood-cerebrospinal fluid barrier, the blood-spinal cord barrier, and the blood-arachnoid barrier. Among them, the blood-brain barrier (BBB) is an obstacle to development of new drugs for the central nervous system diseases. The blood-brain barrier is a physical barrier formed by pericytes and astrocytes surrounding brain capillary endothelial cells connected by tight junctions, and strictly controls the delivery of substances between the peripheral tissues and the brain tissues (Non-Patent Document 2). For example, it is known that substances that are passively diffused from the peripheral tissues to the brain tissues preferably have a molecular weight of 400 g/mol or less and are highly liposoluble. Thus, although many attempts have been made to develop drugs for the central nervous system diseases using low molecular weight compounds, there is concern about side effects due to off-target effects, and drug targets are limited.

In particular, drug targets such as protein-protein interactions that have been attracting attention in recent years have few pockets to which conventional low molecular weight compounds can bind, and middle molecules such as peptides and high molecules such as antibodies are easier to control. The middle and high molecules have high specificity to drug targets, and fewer side effects due to off-target effects can be expected. However, as described above, it is extremely difficult for the middle and high molecules to be passively diffused from the peripheral tissues to the brain tissues due to the presence of the barrier tissues.

As one of methods for delivering the middle or high molecules from the peripheral tissues to the brain tissues, receptor-mediated transcytosis (RMT), transcytosis mediated by a receptor expressed in the blood-brain barrier for delivering specific nutrients or essential components to the brain, has been studied so far. Examples of the receptor include transporters using glucose or amino acids as substrates, and receptors for insulin, transferrin, and lipoprotein (Non-Patent Document 2). There are already commercially available drugs that attempt to deliver drug molecules to the central tissues by conjugating an antibody that binds to a human transferrin receptor with the drug molecules. This indicates that the concept of RMT has been proved by human clinical trials (Non-Patent Document 3).

Low-density lipoprotein receptor-related protein-1 (LRP1) is also one of the receptors that are expected to achieve RMT. The expression level of LRP1 at the blood-brain barrier is well correlated between humans, monkeys, and mice. The amino acid sequences of cluster 2 (CL2) and cluster 4 (CL4), which are ligand-binding domains interacting with diverse endogenous ligands, are very highly conserved across species. Further, molecules obtained by conjugating paclitaxel, which is an anticancer drug, to Angiopep-2 (ANG2) known as a LRP1 binding peptide have completed the second phase of human clinical trials, and RMT via LRP1 in humans has been demonstrated to be safe (Non-Patent Document 4).

### CITATION LIST

### NON-PATENT DOCUMENTS

Non-Patent Document 1: L. Ereshefsky, R. Evans, R. Sood, D. Williamson, B. A. English, "VENTURING INTO A NEW ERA OF CNS DRUG DEVELOPMENT TO IMPROVE SUCCESS" 2015.
Non-Patent Document 2: V. M. Pulgar, Front. Neurosci. 2019, 12: 1019.
Non-Patent Document 3: R Yamamoto and S Kawashima. Nihon Yakugaku Zasshi. 2022, 157(1): 62-75.
Non-Patent Document 4: P Kumthekar, SC. Tang, A. J. Brenner, S. Kesari, D. E. Piccioni, C. Anders, J. Carrillo, P Chalasani, P Kabos, S Puhalla, K Tkaczuk, A. A. Garcia, M. S. Ahluwalia, J. S. Wefel, N. Lakhani and N. Lbrahm. Clin. Cancer. Res. 2020, 26(12): 2789-2799.
Non-Patent Document 5: K Sakamoto, T Shinohara, Y Adachi, T Asami and T Ohtaki. Biochem. Biophys. Rep. 2017, 12: 135-139.
Non-Patent Document 6: F Tanaka, S Dohgu, A Yamauchi, J Matsumoto, T Machida, K Fujishita, K Shibata, Y Shinozaki, K Sato, Y Kataoka, and S Koizumi. PLOS One. 2013 8(1): e55166.
Non-Patent Document 7: D Watanabe, S Nakagawa, Y Morofuji, A Toth, M Vastag, J Aruga, M Niwa, and M Deli. Pharmaceutics. 2021, 13(9): 1484.
Non-Patent Document 8: D P Fairlie and A Dantas. Biopolymers 2016, 106(6): 843-852.
Non-Patent Document 9: Y H Lau, P Andrade, Y Wu, and D R. Spring. Chem Soc Rev. 2015, 44 (1) 91-102.

### SUMMARY OF THE INVENTION

### TECHNICAL PROBLEM

The invention described herein aims to provide a novel peptide that binds to LRP1. The present invention also aims to provide a method for using a "certain molecule" as a pharmaceutical, a diagnostic agent, and/or a research reagent by combining the peptide with the "certain molecule" and delivering the "certain molecule" to brain tissues by RMT via LRP1.

### SOLUTION TO THE PROBLEM

The present inventors have searched for molecules that bind to LRP1, and have achieved the present invention. Specifically, in one aspect, the present invention is directed to a linear or cyclic peptide having an amino acid sequence represented by the following formula (1): X^{N}-X⁵-X⁶-X⁷-X⁸-X⁹-X¹⁰-X¹¹-X¹²-X¹³-X¹⁴-X¹⁵ (1) or a pharmaceutically acceptable salt thereof.

In the formula (1), X^{N} is any of one to four amino acid residues, X⁵ and X¹⁵ each independently represent serine, threonine, cysteine, D-cysteine, or proline, X⁶ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline, X⁷ represents histidine, tyrosine, O-methyl-tyrosine, phenylalanine, 4-amino-phenylalanine, 4-fluoro-phenylalanine, or 4-chloro-phenylalanine, X⁸ represents ornithine, lysine, homolysine, arginine, or homoarginine, X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine, X¹⁰ represents methionine, leucine, norleucine, isoleucine, valine, lysine, or algin, X¹¹ and X¹⁴ each independently represent methionine, leucine, norleucine, isoleucine, valine, 2-aminoheptanoic acid, or 2-aminooctanoic acid, X¹² represents alanine, D-alanine, or 2-aminoisobutyric acid, X¹³ represents glycine, alanine, asparagine, aspartic acid, glutamine, or glutamic acid, an N-terminal amino group and a C-terminal carboxy group is optionally modified or deleted, and X⁵ and X¹⁵ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (1).

Preferred embodiments of the amino acid residues from X¹ to X¹⁵ in the peptide of the formula (1), which will be described later in detail in the embodiments, can be arbitrarily combined independently of each other.

More specifically, the present invention includes the following embodiments.
[1] A linear or cyclic peptide having an amino acid sequence represented by the formula (1): X^{N}-X⁵-X⁶-X⁷-X⁸-X⁹-X¹⁰-X¹¹-X¹²-X¹³-X¹⁴-X¹⁵ (1) (wherein X^{N} is any of one to four amino acid residues, X⁵ and X¹⁵ each independently represent serine, threonine, cysteine, D-cysteine, or proline, X⁶ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline, X⁷ represents histidine, tyrosine, O-methyl-tyrosine, phenylalanine, 4-amino-phenylalanine, 4-fluoro-phenylalanine, or 4-chloro-phenylalanine, X⁸ represents ornithine, lysine, homolysine, arginine, or homoarginine, X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine, X¹⁰ represents methionine, leucine, norleucine, isoleucine, valine, lysine, or algin, X¹¹ and X¹⁴ each independently represent methionine, leucine, norleucine, isoleucine, valine, 2-aminoheptanoic acid, or 2-aminooctanoic acid, X¹² represents alanine, D-alanine, or 2-aminoisobutyric acid, X¹³ represents glycine, alanine, asparagine, aspartic acid, glutamine, or glutamic acid, an N-terminal amino group and a C-terminal carboxy group is optionally modified or deleted, and X⁵ and X¹⁵ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (1)) or a pharmaceutically acceptable salt thereof.
[2] A linear or cyclic peptide having an amino acid sequence represented by the following formula (3): X^{N}-X⁵-X⁶-X⁷-X⁸-X⁹-X¹⁰-X¹¹-X¹²-X¹³-X¹⁴-Pro-X¹⁶ (3) (wherein X^{N} is any of one to four amino acid residues, X⁵ and X¹⁶ each independently represent serine, threonine, cysteine, D-cysteine, or proline, X⁶ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline, X⁷ represents histidine, tyrosine, O-methyl-tyrosine, phenylalanine, 4-amino-phenylalanine, 4-fluoro-phenylalanine, or 4-chloro-phenylalanine, X⁸ represents ornithine, lysine, homolysine, arginine, or homoarginine, X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine, X¹⁰ represents methionine, leucine, norleucine, isoleucine, valine, lysine, or algin, X¹¹ and X¹⁴ each independently represent methionine, leucine, norleucine, isoleucine, valine, 2-aminoheptanoic acid, or 2-aminooctanoic acid, X¹² represents alanine, D-alanine, or 2-aminoisobutyric acid, X¹³ represents glycine, alanine, asparagine, aspartic acid, glutamine, or glutamic acid, an N-terminal amino group and a C-terminal carboxy group is optionally modified or deleted, and X⁵ and X¹⁶ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (3)) or a pharmaceutically acceptable salt thereof.
[3] A linear or cyclic peptide having an amino acid sequence represented by the following formula (3): X^{N}-X⁵-X⁶-X⁷-X⁸-X⁹-X¹⁰-X¹¹-X¹²-X¹³-X¹⁴-Pro-X¹⁶ (3) (wherein X^{N} is any of one to four amino acid residues, X⁶ and X¹⁶ each independently represent serine, threonine, cysteine, D-cysteine, or proline, X⁵ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline, X⁷ represents histidine, tyrosine, O-methyl-tyrosine, phenylalanine, 4-amino-phenylalanine, 4-fluoro-phenylalanine, or 4-chloro-phenylalanine, X⁸ represents ornithine, lysine, homolysine, arginine, or homoarginine, X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine, X¹⁰ represents methionine, leucine, norleucine, isoleucine, valine, lysine, or algin, X¹¹ and X¹⁴ each independently represent methionine, leucine, norleucine, isoleucine, valine, 2-aminoheptanoic acid, or 2-aminooctanoic acid, X¹² represents alanine, D-alanine, or 2-aminoisobutyric acid, X¹³ represents glycine, alanine, asparagine, aspartic acid, glutamine, or glutamic acid, an N-terminal amino group and a C-terminal carboxy group is optionally modified or deleted, and X⁶ and X¹⁶ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (3)), or a pharmaceutically acceptable salt thereof.
[4] The linear or cyclic peptide or pharmaceutically acceptable salt thereof, of any one of [1] to [3], wherein X^{N} is Lys-Gly-Thr-Pro or Gly-Thr-Pro.
[5] A linear or cyclic peptide or pharmaceutically acceptable salt thereof, of any one of [1] to [4], wherein the amino acid sequence has one or several amino acids deleted, added, and/or substituted and has LRP1 binding activity.
[6] A derivative and/or modified form of the peptide of any one of [1] to [5] and the following [14] to [21].
[7] The derivative or modified form of the peptide of [6], wherein the derivative or the modified form has an azide group, an alkyne group, a cyclooctyne group, or a tetrazine group bound to the N-terminus, the C-terminus, or an amino acid side chain directly or via a linker.
[8] A pharmaceutical, a diagnostic agent, and/or a reagent including the peptide of any one of [1] to [7] and the following [14] to [21] or a derivative or modified form thereof.
[9] A peptide-drug conjugate including the derivative or modified form of the peptide of [6] and a drug linked together directly or via a linker.
[10] The peptide-drug conjugate of [9], wherein the drug is an antibody, a nucleic acid, or a bioactive peptide.
[11] A polynucleotide encoding an amino acid sequence consisting of only natural amino acids in the amino acid sequence of any one of [1] to [5] or the following [14] to [21], or a polynucleotide encoding a peptide having a base sequence with a sequence identity of 70% or more to the polynucleotide and having LRP1 binding activity.
[12] An expression vector including the polynucleotide of [11].
[13] A cell, yeast, bacterium, virus, liposome, or nanoparticle that presents the amino acid sequence of any one of [1] to [5] and the following [14] to [21].
[14] A linear and cyclic peptide having an amino acid sequence represented by the following formula (6): X¹-X²-X³-X⁴-X^{L}-X⁷-X⁸-X⁹-X¹⁰-X¹¹-X¹²-X¹³-X¹⁴-X^{M} (6) (wherein X^{L} is X⁵-X⁶, X^{M} is X¹⁵-X¹⁶, X^{L} and X^{M} each independently represent an amino acid residue having an amino group, a carboxy group, a thiol group, an allyl group, an alkynyl group, an azide group, or a halogen atom, or a derivative thereof when involved in cyclization in a peptide molecule, X⁵, X⁶, and X¹⁵ each independently represent any amino acid residue or a derivative thereof and X¹⁶ represents any amino acid residue, a derivative thereof, or a deletion when not involved in cyclization in the peptide molecule, X⁷ represents an amino acid residue having a hydrocarbon group that optionally has a substituent, an amino acid residue having an aromatic carbocyclic group that optionally has a substituent, an amino acid residue having an aromatic heterocyclic group that optionally has a substituent, or a derivative thereof, X⁸ represents a positively charged amino acid residue or a derivative thereof, X¹¹, X¹² and X¹⁴ each independently represent an amino acid residue having a hydrocarbon group that optionally has a substituent or a derivative thereof, X¹³ represents an amino acid residue having a hydrocarbon group that optionally has a substituent, a negatively charged amino acid residue, or a derivative thereof, X⁹ and X¹⁰ each independently represent any amino acid residue or a derivative thereof, X¹, X², X³, and X⁴ each independently represent any amino acid residue, a derivative thereof, or a deletion, a covalent bond formed between X^{L} and X^{M} directly or indirectly via a linker optionally provides one cyclic structure in a molecule of the peptide of the formula (6), the covalent bond between X^{L} and X^{M} being any of a main chain to main chain bond, a main chain to side chain bond, a side chain to main chain bond, or a side chain to side chain bond, and an N-terminal amino group and a C-terminal carboxy group is optionally modified or deleted), or a pharmaceutically acceptable salt thereof.
[15] The linear and cyclic peptide or pharmaceutically acceptable salt thereof, of [14], wherein a bond between Cα-carbon atoms of X⁵ and X¹⁵, a bond between Cα-carbon atoms of X⁶ and X¹⁵, a bond between Cα-carbon atoms of X⁵ and X¹⁶, and a bond between Cα-carbon atoms of X⁶ and X¹⁶ are each independently represented by the following formula (7): Cα^{L}-(CH₂)_{A}-Z-(CH₂)_{B}-Cα^{M} (7) (wherein L is 4 or 5, M is 14 or 15, A and B each independently represent any integer of 0 to 2, the sum of A and B is any integer of 0 to 4, and Z is S-S, CH₂-CH₂, S-CH₂, CH₂-S, O-CH₂, CH₂-O, CH=CH, NH-C(=O), N(CH₃)-C(=O), NH-C(=S), N(CH₃)-C(=S), O-C(=O), C(=O)-NH, C(=O)-N(CH₃), C(=S)-NH, C(=S)-N(CH₃), C(=O)-O, CH₂-CH₂-CH₂, S-CH₂-CH₂, CH₂-CH₂-S, CH₂-S-CH₂, O-CH₂-CH₂, CH₂-CH₂-O, CH₂-O-CH₂, S-CH₂-S, S-C(=CH₂)-S, S-(C(=O)-CH₃)-S, S-(CH₂)₂-S, S-(CH₂)₃-S, S-CH₂-C(=O)-CH₂-S, S-CH₂-C(=CH₂)-CH₂-S, S-(CH₂)₄-S, S-CH₂-CH=CH-CH₂-S, S-CH₂-C₆H₄-CH₂-S (a methylene group is optionally bound to a phenylene ring at any of ortho, meta or para), S-CH₂-C(=O)-CH₂-S, or S-CH₂-C(=CH₂)-CH₂-S).
[16] The linear and cyclic peptide or pharmaceutically acceptable salt of [14] or [15], wherein X₈ is 2,3-diaminopropionic acid, 2,4-diaminobutanoic acid, ornithine, lysine, homolysine, arginine, homoarginine, D-amino acid thereof, N-methylated amino acid thereof, α-methylated amino acid thereof, or a derivative thereof.
[17] The linear and cyclic peptide or pharmaceutically acceptable salt thereof, of any one of [14] to [16], wherein X¹² is alanine, D-alanine, 2-aminoisobutyric acid, 2-aminobutanoic acid, D-2-aminobutanoic acid, isovaline, D-isovaline, valine, D-valine, isoleucine, D-isoleucine, leucine, D-leucine, N-methylated amino acid thereof, α-methylated amino acid thereof, or a derivative thereof.
[18] The linear and cyclic peptide or pharmaceutically acceptable salt thereof, of any one of [14] to [17], wherein X¹¹ and X¹⁴ are each independently methionine, an amino acid residue represented by the formula (8): 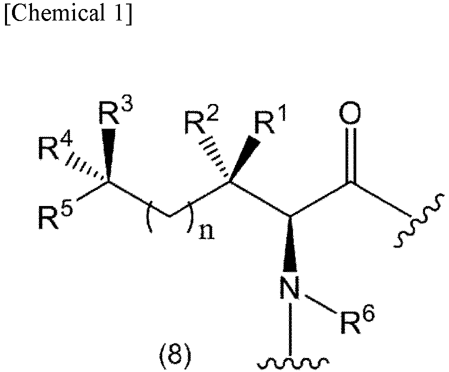 (wherein a wavy line represents a point of attachment to a carbonyl group or a nitrogen atom forming an amide bond of a main chain, R¹, R², and R⁶ each independently represent a hydrogen atom or a methyl group, R³, R⁴, and R⁵ each independently represent a hydrogen atom, a methyl group, or a halogen atom (F, Cl, Br, I), and n represents any integer of 0 to 10);
   the formula (9): 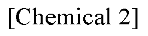 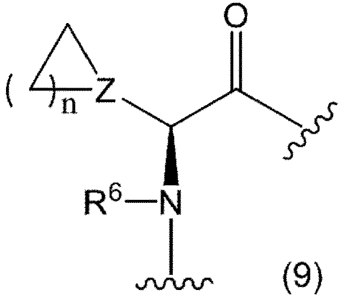 (wherein Z represents C or N, R⁶ represents a hydrogen atom or a methyl group, and n represents any integer of 1 to 6); or
   the formula (10): 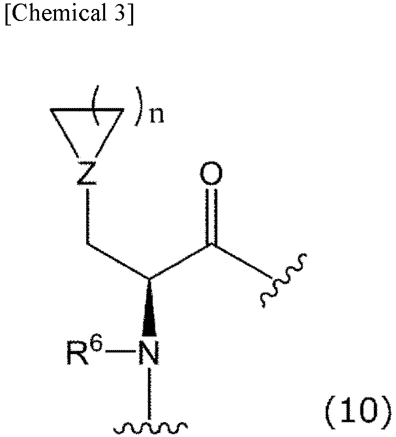 (wherein a wavy line represents a site of bonding to a carbonyl group or a nitrogen atom forming an amide bond of a main chain, Z represents C or N, R⁶ represents a hydrogen atom or a methyl group, and n represents any integer of 1 to 6), or a derivative thereof.
[19] The linear and cyclic peptide or pharmaceutically acceptable salt thereof, of any one of [14] to [18], wherein X⁷ is represented by the formula (8): 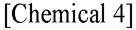 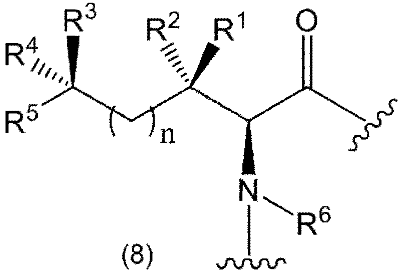 (wherein a wavy line represents a point of attachment to a carbonyl group or a nitrogen atom forming an amide bond of a main chain, R¹, R², and R⁶ each independently represent a hydrogen atom or a methyl group, R³, R⁴, and R⁵ each independently represent a hydrogen atom, a methyl group, or a halogen atom (F, Cl, Br, I), and n represents any integer of 0 to 10);
   the formula (9): 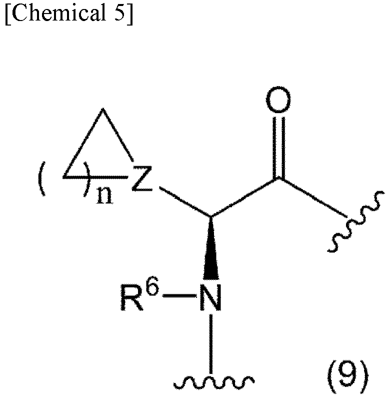 (wherein Z represents C or N, R⁶ represents a hydrogen atom or a methyl group, and n represents any integer of 1 to 6);
   the formula (10): 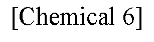 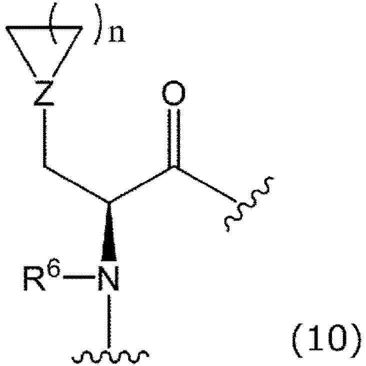 (wherein a wavy line represents a site of bonding to a carbonyl group or a nitrogen atom forming an amide bond of a main chain, Z represents C or N, R⁶ represents a hydrogen atom or a methyl group, and n represents any integer of 1 to 6);
   the formula (11): 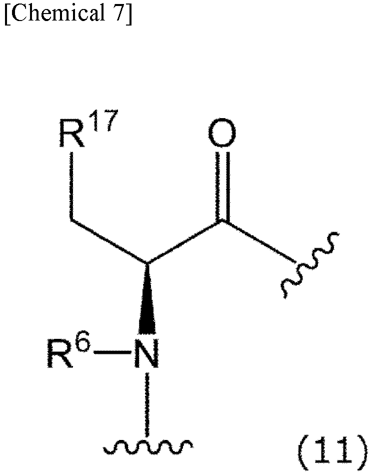 (wherein R¹² represents a 2-thienyl group, a 3-thienyl group, a 2-pyridyl group, a 3-pyridyl group, a 4-pyridyl group, a 2-thiazolyl group, a 4-thiazolyl group, a 2-oxazolyl group, a 4-oxazolyl group, a 1-pyrazolyl group, a 1H-imidazol-4-yl group, a 1-imidazolyl group, a 1,2,3-triazol-1-yl group, or a 1,2,4-triazol-1-yl group, and R⁶ represents a hydrogen atom or a methyl group);
   the formula (12): 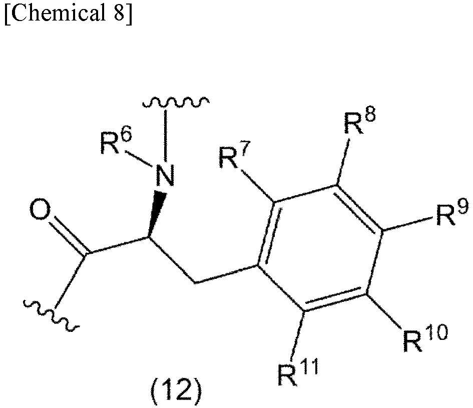 (wherein a wavy line represents a point of attachment to a carbonyl group or a nitrogen atom forming an amide bond of a main chain, R⁷, R⁸, R⁹, R¹⁰, and R¹¹ each independently represent a hydrogen atom, a hydroxy group, a methoxy group, a tert-butyl group, a halogenated methyl group, or a halogen atom (F, Cl, Br, I), and R⁶ represents a hydrogen atom or a methyl group); or
   the formula (13): 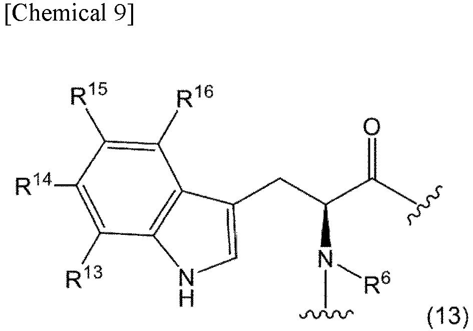 (wherein R¹³, R¹⁴, R¹⁵, and R¹⁶ each independently represent a hydrogen atom, a hydroxy group, a methoxy group, a methyl group, a tert-butyl group, a halogenated methyl group, or a halogen atom (F, Cl, Br, I), and R⁶ represents a hydrogen atom or a methyl group), or a derivative thereof.
[20] The linear and cyclic peptide or pharmaceutically acceptable salt thereof, of any one of [14] to [19], wherein X¹³ is glycine, alanine, aspartic acid, glutamic acid, asparagine, glutamine, D-alanine, D-aspartic acid, D-glutamic acid, D-asparagine, D-glutamine, N-methylated amino acid thereof, α-methylated amino acid thereof, or a derivative thereof.
[21] The linear and cyclic peptide or pharmaceutically acceptable salt thereof, of any one of [14] to [20], wherein X^{L} and X^{M} which are not involved in cyclization in a peptide molecule are each independently alanine, isovaline, norvaline, serine, homoserine, threonine, allothreonine, O-methylated serine, O-methylated homoserine, O-methylated threonine, O-methylated allothreonine, 2,3-diaminopropionic acid, 2,4-diaminobutanoic acid, ornithine, lysine, arginine, proline, cis-4-hydroxy-proline, trans-4-hydroxy-proline, azetidine-2-carboxylic acid, pipecolic acid, D-serine, D-homoserine, D-threonine, D-allothreonine, D-O-methylated serine, D-O-methylated homoserine, D-O-methylated threonine, D-O-methylated allothreonine, D-2,3-diaminopropionic acid, D-2,4-diaminobutanoic acid, D-ornithine, D-lysine, D-arginine, D-proline, D-cis-4-hydroxy-proline, D-trans-4-hydroxy-proline, D-azetidine-2-carboxylic acid, D-pipecolic acid, N-methylated amino acid thereof, α-methylated amino acid thereof, or a derivative thereof.

### ADVANTAGES OF THE INVENTION

The present invention provides a linear and cyclic peptide that is novel and useful for delivering a "certain molecule" combined with the peptide to brain tissues by RMT via LRP1 to which the peptide is bound so that the "certain molecule" is used as a pharmaceutical, a diagnostic agent, and/or a research reagent.

### BRIEF DESCRIPTION OF THE DRAWINGS

FIG. 1 shows the results of evaluation of concentration-dependent binding activities of Sequences 45 and 46, which are representative examples of biotin-labeled peptides of the present invention, to LRP1 cluster 2 (CL2)-Fc chimeric proteins, LRP1 cluster 3 (CL3)-Fc chimeric proteins, and LRP1 cluster 4 (CL4)-Fc chimeric proteins. For the respective proteins, the concentrations of the biotin-labeled peptides used in the experiment are (1) 1,000 nM, (2) 330 nM, (3) 110 nM, (4) 37 nM, (5) 12 nM, (6) 4.1 nM, (7) 1.4 nM, and 0 nM in order from the left, and are shown by shading the bars.
FIG. 2 schematically shows a competitive binding assay by ELISA used for evaluating the peptide of the present invention for both the binding activity to LRP1 cluster 4 and the resistance to protease degradation. SA represents streptavidin, B represents biotin, and HRP represents horseradish peroxidase.
FIG. 3 shows the results of evaluating the binding activity (Binding (%)) of Sequence 46 (500 nM), which is a representative example of the biotin-labeled peptide of the present invention, to LRP1 cluster 4-Fc chimeric proteins in the presence of Sequence 2 (2,000 to 63 nM) immediately or 24 hours after mixing with mouse plasma. The peptide concentrations of Sequence 2 are (1) 2,000 nM, (2) 1,000 nM, (3) 500 nM, (4) 250 nM, (5) 130 nM, and (6) 63 nM in order from the left and are shown by shading the bars.
FIG. 4 shows the results of evaluating the inhibition activity (Inhibition (%)) of Sequence 46 (500 nM), which is a representative example of the biotin-labeled peptide of the present invention, to LRP1 cluster 4-Fc chimeric proteins in the presence of Sequences 1 to 44 (2,000 nM) immediately or 24 hours after mixing with mouse plasma.
FIG. 5 is a schematic view of an in vitro blood-brain barrier model for assessing the permeability through the blood-brain barrier.
FIG. 6 shows the results of evaluation of the ability of Sequence 2, a representative example of the peptide of the present invention labeled with 5-FAM (fluorescent dye), to pass through the blood-brain barrier using in vitro blood-brain barrier models (rat and monkey).
FIG. 7 shows the structural formulae of Sequences 2 and 46, which are representative examples of the peptide of the present invention used in Examples.
FIG. 8 shows the results of RP-HPLC analysis of DBCO-KS-487, a modified form of the peptide of the present invention (before reaction), and a solution of the modification reacted with 6-azidohexanoic acid at room temperature (after reaction).

### DESCRIPTION OF EMBODIMENTS

Embodiments of the present invention will be described below with reference to the drawings. The embodiments described below are each not limited to the invention according to claims. All components described in the embodiments and combinations thereof are not necessarily essential to solution according to the present invention.

### (Definitions)

As used herein, a peptide refers to two or more amino acids bound together by amide bonds (peptide bonds), and can be, for example, 2 to 20 amino acids bound together by amide bonds. The left end is the N-terminus (amino terminus) and the right end is the C-terminus (carboxy terminus) according to the peptide notation. The first carbon atom adjacent to the carbonyl group forming the peptide bond is referred to as Cα carbon.

As used herein, "any amino acid residue or derivative thereof" is used in its broadest sense and encompasses, in addition to natural amino acids, artificial amino acids having non-natural structures, chemically synthesized compounds having properties that are characteristics of amino acids known in the art, and also carboxylic acids having functional groups. Examples of unnatural amino acids include, but are not limited to, a D-amino acid, an α/α-disubstituted amino acid (e.g., α-methylated amino acids such as 2-aminoisobutyric acid) with a main chain structure different from those of natural amino acids, an N-alkyl-amino acid (e.g., N-methylated amino acid), an N-substituted glycine (peptoid), an amino acid (e.g., β homoamino acid and γ homoamino acid) with an extended main chain, an amino acid with a side chain structure different from those of natural amino acids (e.g., cyclohexylalanine, allylglycine, 2-(2-pyridyl)-glycine, and 3-(1H-benzimidazol-2-yl)-alanine), an amino acid (e.g., norleucine, diaminopropanoic acid, and 3-(2-pyridyl)-alanine) with a partially substituted side chain, an amino acid with an extra functional group in a side chain; an amino acid (e.g., homonorleucine and γ-methylleucine) with an extra C, alkyl group, or a methyl group in a side chain, an amino acid (e.g., 3-chloro-alanine) with a halogen atom (F, Cl, Br, I) in a side chain, a carboxylic acid (3-chloropropionic acid) with a halogen atom (F, Cl, Br, I) in a side chain, a carboxylic acid (e.g., 3-butenoic acid) with a functional group in a side chain, an amino acid (e.g., β-azidoalanine, ornithine) with an extra N or an amino group in a side chain, an amino acid with an extra O or a methoxy group in a side chain (e.g., O-methyl-serine, O-methyl-threonine), an amino acid (e.g., 3-hydroxy-phenylalanine) with an extra hydroxy group in a side chain, an amino acid (e.g., 3-carboxy-phenylalanine) with an extra carboxy group (-COOH) in a side chain, an amino acid (e.g., ethionine) with an extra S in a side chain, an amino acid (e.g., aspartic acid-4-methyl ester) in which a carboxylic acid functional group in a side chain is protected by esters, and an amino acid (methionine sulfoxide) in which a thio group (-S-) in a side chain has been oxidized and converted to a sulfinyl group (-S(=O)-) or a sulfonyl group (-S(=O)₂-).

As used herein, "LRP1" means LRP1 of mammals such as mice, rats, dogs, monkeys, and humans.

As used herein, the phrase "having LRP1 binding activity" means that the peptide of the present invention or a combination of the peptide of the present invention and a "certain molecule" binds to full-length protein of LRP1, partial protein such as cluster 4, or a chimera of these proteins and other proteins in a concentration-dependent manner in an in vitro test. The presence or absence of the LRP1 binding activity can be checked by any known method by those skilled in the art with reference to Non-Patent Document 5, for example.

As used herein, a "barrier tissue" refers to the barrier tissue of mammals such as mice, rats, dogs, monkeys, and humans.

As used herein, the phrase "pass through the barrier tissue" means that: (1) the peptide of the present invention or a combination of the peptide of the present invention and a "certain molecule" moves from a blood vessel well to brain well of a blood-brain barrier model used in an in vitro test; (2) the peptide of the present invention or the combination of the peptide of the present invention and the "certain molecule" administered from the periphery is detected in the brain tissue in a pharmacokinetic test by LC-MS/MS; and (3) the peptide of the present invention or the combination of the peptide of the present invention and the "certain molecule" administered from the periphery is detected in the brain in a pharmacokinetic test using an in vivo imaging system. If at least one of the definitions (1) to (3) is satisfied, it can be said that the peptide or the combination "passes through the barrier tissue." Whether the peptide or the combination has the ability to pass through the barrier tissue can be checked by any known method by those skilled in the art with reference to Non-Patent Documents 6 and 7, for example.

### (Peptide of the Present Invention)

[1] A linear and cyclic peptide of an embodiment of the present disclosure has an amino acid sequence represented by the following formula (1): X^{N}-X⁵-X⁶-X⁷-X⁸-X⁹-X¹⁰-X¹¹-X¹²-X¹³-X¹⁴-X¹⁵ (1). In the formula (1), X^{N} is any of one to four amino acid residues, X⁵ and X¹⁵ each independently represent serine, threonine, cysteine, D-cysteine, or proline, X⁶ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline, X⁷ represents histidine, tyrosine, O-methyl-tyrosine, phenylalanine, 4-amino-phenylalanine, 4-fluoro-phenylalanine, or 4-chloro-phenylalanine, X⁸ represents ornithine, lysine, homolysine, arginine, or homoarginine, X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine, X¹⁰ represents methionine, leucine, norleucine, isoleucine, valine, lysine, or algin, X¹¹ and X¹⁴ each independently represent methionine, leucine, norleucine, isoleucine, valine, 2-aminoheptanoic acid, or 2-aminooctanoic acid, X¹² represents alanine, D-alanine, or 2-aminoisobutyric acid, X¹³ represents glycine, alanine, asparagine, aspartic acid, glutamine, or glutamic acid. An N-terminal amino group and a C-terminal carboxy group is optionally modified or deleted, and X⁵ and X¹⁵ may be cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (1).

In the peptide of the formula (1), X^{N} is preferably Lys-Gly-Thr-Pro or Gly-Thr-Pro. When lysine is at the N-terminus, the peptide is optionally bound to a certain molecule via an ε-amino group of the side chain. When glycine is at the N-terminus, the peptide is optionally bound to a certain molecule via an α-amino group. It is preferable that X⁵ and X¹⁵ are cysteine or D-cysteine and form a covalent bond via a linker such as a disulfide bond or a methylene group between the side chain and the SH group, providing a cyclic structure in the molecule.

More preferred embodiments of the peptide of the above formula (1) includes an amino acid sequence represented by the following formula (2). The amino acid sequence may form a covalent bond between the side chain and the SH group via a linker such as a disulfide bond or a methylene group, forming a cyclic structure in the molecule, or is optionally bound to a certain molecule via an ε-amino group of Lys1.

Ac-Lys-Gly-Thr-Pro-Cys-X⁶-X⁷-Lys-X⁹-X¹⁰-Leu-Ala-Glu-X¹⁴-Cys-OH (2)

In the formula (2), X⁶ represents serine, threonine, or 2,3-diaminopropionic acid, X⁷ represents histidine, tyrosine, phenylalanine, or tryptophan, X⁹ represents tyrosine, methionine, norleucine, or arginine, X¹⁰ represents methionine, norleucine, arginine, or lysine, and X¹⁴ represents leucine, isoleucine, methionine, or norleucine.

[2] The linear and cyclic peptide of another embodiment of the present disclosure has an amino acid sequence represented by the following formula (3): X^{N}-X⁵-X⁶-X⁷-X⁸-X⁹-X¹⁰-X¹¹-X¹²-X¹³-X¹⁴-Pro-X¹⁶ (3). In the formula (3), X^{N} is any of one to four amino acid residues, X⁵ and X¹⁶ each independently represent serine, threonine, cysteine, D-cysteine, or proline, X⁶ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline, X⁷ represents histidine, tyrosine, O-methyl-tyrosine, phenylalanine, 4-amino-phenylalanine, 4-fluoro-phenylalanine, or 4-chloro-phenylalanine, X⁸ represents ornithine, lysine, homolysine, arginine, or homoarginine, X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine, X¹⁰ represents methionine, leucine, norleucine, isoleucine, valine, lysine, or algin, X¹¹ and X¹⁴ each independently represent methionine, leucine, norleucine, isoleucine, valine, 2-aminoheptanoic acid, or 2-aminooctanoic acid, X¹² represents alanine, D-alanine, or 2-aminoisobutyric acid, X¹³ represents glycine, alanine, asparagine, aspartic acid, glutamine, or glutamic acid. An N-terminal amino group and a C-terminal carboxy group is optionally modified or deleted, and X⁵ and X¹⁶ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (3).

In the peptide of the above formula (3), X^{N} is preferably Lys-Gly-Thr-Pro or Gly-Thr-Pro. When lysine is at the N-terminus, the peptide is optionally bound to a certain molecule via an ε-amino group of the side chain. When glycine is at the N-terminus, the peptide is optionally bound to a certain molecule via an α-amino group. It is preferable that X⁵ and X¹⁶ are cysteine or D-cysteine and form a covalent bond between the side chain and the SH group via a linker such as a disulfide bond or a methylene group, forming a cyclic structure in the molecule.

More preferred embodiments of the peptide of the above formula (3) includes an amino acid sequence represented by the following formula (4). The amino acid sequence may form a covalent bond between the side chain and the SH group via a linker such as a disulfide bond or a methylene group, forming a cyclic structure in the molecule, or is optionally bound to a certain molecule via an ε-amino group of Lys1.

Ac-Lys-Gly-Thr-Pro-Cys-X⁶-X⁷-Lys-X⁹-X¹⁰-Leu-Ala-Glu-X¹⁴-Pro-Cys-OH (4)

In the formula (4), X⁶ represents serine, threonine, or 2,3-diaminopropionic acid, X⁷ represents histidine, tyrosine, phenylalanine, or tryptophan, X⁹ represents tyrosine, methionine, norleucine, or arginine, X¹⁰ represents methionine, norleucine, arginine, or lysine, and X¹⁴ represents leucine, isoleucine, methionine, or norleucine.

[3] A linear and cyclic peptide of another embodiment of the present disclosure has an amino acid sequence represented by the following formula (3): X^{N}-X⁵-X⁶-X⁷-X⁸-X⁹-X¹⁰-X¹¹-X¹²-X¹³-X¹⁴-Pro-X¹⁶ (3). In the formula (3), X^{N} is any of one to four amino acid residues, X⁶ and X¹⁶ each independently represent serine, threonine, cysteine, D-cysteine, or proline, X⁵ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline, X⁷ represents histidine, tyrosine, O-methyl-tyrosine, phenylalanine, 4-amino-phenylalanine, 4-fluoro-phenylalanine, or 4-chloro-phenylalanine, X⁸ represents ornithine, lysine, homolysine, arginine, or homoarginine, X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine, X¹⁰ represents methionine, leucine, norleucine, isoleucine, valine, lysine, or algin, X¹¹ and X¹⁴ each independently represent methionine, leucine, norleucine, isoleucine, valine, 2-aminoheptanoic acid, or 2-aminooctanoic acid, X¹² represents alanine, D-alanine, or 2-aminoisobutyric acid, X¹³ represents glycine, alanine, asparagine, aspartic acid, glutamine, or glutamic acid, an N-terminal amino group and a C-terminal carboxy group is optionally modified or deleted, and X⁶ and X¹⁶ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (3).

In the peptide of the above formula (3), X^{N} is preferably Lys-Gly-Thr-Pro or Gly-Thr-Pro. When lysine is at the N-terminus, the peptide is optionally bound to a certain molecule via an ε-amino group of the side chain. When glycine is at the N-terminus, the peptide is optionally bound to a certain molecule via an α-amino group. It is preferable that X⁶ and X¹⁶ are cysteine or D-cysteine and form a covalent bond between the side chain and the SH group via a linker such as a disulfide bond or a methylene group, forming a cyclic structure in the molecule.

More preferred embodiments of the peptide of the above formula (3) includes an amino acid sequence represented by the following formula (5). The amino acid sequence may form a covalent bond between the side chain and the SH group via a linker such as a disulfide bond or a methylene group, forming a cyclic structure in the molecule, or is optionally bound to a certain molecule via an ε-amino group of Lys1.

Ac-Lys-Gly-Thr-Pro-X⁵-Cys-X⁷-Lys-X⁹-X¹⁰-Leu-Ala-Glu-X¹⁴-Pro-Cys-OH (5)

In the formula (5), X⁵ represents serine, threonine, or 2,3-diaminopropionic acid, X⁷ represents histidine, tyrosine, phenylalanine, or tryptophan, X⁹ represents tyrosine, methionine, norleucine, or arginine, X¹⁰ represents methionine, norleucine, arginine, or lysine, and X¹⁴ represents leucine, isoleucine, methionine, or norleucine.

[4] The peptide according to the present invention encompasses a peptide having binding activity to VRP1 even if the peptide has homology in which one to several amino acids are deleted, added and/or substituted in any of the amino acid sequences [1] to [3]. As used herein, the "peptide in which one to several amino acids are deleted, added, and/or substituted" optionally has any number of amino acids deleted, added, and/or substituted as long as the peptide has the LRP1 binding activity, but the number is preferably one to five, more preferably one or two. The deletion, addition, and/or substitution may occur at either the end or the middle of the peptide, and may occur at one or more locations.

Examples of the amino acid sequence in which one to several amino acids are deleted, added and/or substituted in the amino acid sequence include those having at least 50% or more, preferably 70% or more, more preferably 80% or more, particularly preferably 90% or more of the identity with the amino acid sequence when calculated using Basic Local Alignment Search Tool of National Center for Biological Information (BLAST) (e.g., by using default or initial setup parameters) or the like.

It is also known that there are peptides and protein domains that are less homologous in primary structure but are very similar in three-dimensional structure. Thus, peptides having at least 50% or more, preferably 70% or more, more preferably 80% or more, and particularly preferably 90% or more homology in three-dimensional structure to the above-mentioned amino acid sequence and having Ras binding activity are also included in the peptide of the present embodiment. The homology in three-dimensional structure between the peptides can be determined by predicting the three-dimensional structures of the peptides in the following manner from amino acid sequences of peptides having unknown three-dimensional structures by using, for example, a homology modeling method. For example, when an amino acid sequence (target sequence) similar to the sequence of the cyclic peptide of the present embodiment (reference peptide) is given, an alignment (aligned sequences) between the target sequence and the reference sequence is given. When an alignment calculated by FASTA, PSI-BLAST, or LIBRA is used, the correspondence between the target sequence and the reference sequence for each amino acid is determined, and the three-dimensional coordinates for each amino acid on the target sequence are generated from the three-dimensional coordinates of the reference peptide based on this correspondence. In the construction of the three-dimensional coordinates, structurally inappropriate gaps, collisions, and distortions may occur between the amino acid residues. Thus, energy minimization calculation is performed to eliminate these structural distortions. Some modeling software does not perform the calculation simultaneously for all the atoms of the peptide, but performs the calculation in stages for smooth elimination of the structural distortions. Specifically, the first calculation is performed for α-carbon atoms forming the backbone of the peptide, the second calculation is performed for the main chain atoms including the α-carbon atoms, and then the final calculation is performed for the whole peptide including the side chain atoms. When the alignment with respect to the target sequence is obtained in this manner, the three-dimensional structure can be predicted and constructed. As the index of three-dimensional structural homology, the root mean square deviation (RMSD), which is a difference between XYZ-coordinates of the three-dimensional structures optimally superimposed, can be used for comparison.

The peptide of the present embodiment also includes a salt of the peptide. As the salt of the peptide, a salt with a physiologically acceptable base or acid is used, and examples thereof include an addition salt of an inorganic acid (such as hydrochloric acid, hydrobromic acid, hydroiodic acid, sulfuric acid, phosphoric acid), an addition salt of an organic acid (such as p-toluenesulfonic acid, methanesulfonic acid, oxalic acid, p-bromophenylsulfonic acid, carboxylic acid, succinic acid, citric acid, benzoic acid, acetic acid), an inorganic base (such as ammonium hydroxide, an alkali or alkaline-earth metal hydroxide, a carbonate, a bicarbonate), and an addition salt of an amino acid.

### (Derivative and Modified Form of Peptide)

The peptide of the present embodiment also includes various peptide derivatives and/or peptide modified forms as long as the object of the present invention is achieved. Examples of such derivatives include: a peptide in which a saturated fatty chain is substituted with an unsaturated fatty chain; a peptide in which some of the atoms are substituted with other atoms including radioactive or non-radioactive isotope atoms; a peptide in which an amide bond is substituted with a thioamide bond (-NH-C(=S)-); a peptide in which an amide bond is substituted with alkene (-C=C-); a peptide in which an amide bond is substituted with alkyl (-C-C-); a peptide in which an amide bond is substituted with hydroxyethylene (-C(-OH)-C-); a peptide in which an amide bond is substituted with ester (-O-C(=O)-); a peptide in which an amide bond is substituted with alkene (-C=C-); a peptide in which an amide bond is substituted with (-C-NH-); and a peptide in which an amide bond is substituted with (-C(=O)-C). Examples of such modified forms include, but are not limited to, a peptide in which a carbon at the α-position is disubstituted; a peptide in which an amide bond is N-alkylated; a peptide in which some of functional groups are modified such as halogenated, cyanated, nitrated, hydroformylated, hydroxylated, aminated, deaminated, dehydroated, amidated, acetylated, methoxylated, prenylated, or alkylated (e.g., a peptide in which some of amino groups are acetylated, alkylated, or deaminated, and a peptide in which some of carboxy groups are amides or esters); a peptide in which S is sulfoxide S(=O) or sulfone S(=O)₂; a peptide that is multimerized via a chemical linker; a peptide that is biotin-labeled; a peptide that is fluorescence-labeled; a peptide that is luminescence-labeled; and a peptide that is fused or conjugated directly or via a linker with an alkyl chain, polyethylene glycol, an antibody, a lectin, a sugar chain, an enzyme, a peptide, a peptoid, a membrane penetrating peptide, a low molecular weight compound, a nucleic acid, and a molecule that induces ubiquitination of a protein.

In some embodiments of the present disclosure, the peptide of the present invention includes an amino acid residue having an azide group at the N- or C-terminus. Thus, a "certain molecule" having an alkyne group can be covalently bound to the N- or C-terminus of the peptide of the present invention by "copper (I)-catalyzed alkyne-azide cycloaddition (CuAAC) reaction" (or "click" reaction for short). In another embodiment of the present disclosure, a linker having an azide group and an N-hydroxysuccinimide (NHS) ester group is reacted with the α- or ε-amino group of the peptide of the present invention to introduce the azide group. Thus, the "certain molecule" having the alkyne group can be covalently bound to the peptide of the present invention by the CuAAC reaction. In another embodiment of the present disclosure, the peptide of the present invention includes an amino acid residue having an alkyne group at the N- or C-terminus. Thus, a "certain molecule" having an azide group can be covalently bound to the N- or C-terminus of the peptide of the present invention by the CuAAC reaction. **In** another embodiment of the present disclosure, a linker having an alkyne group and an NHS ester group is reacted with the α- or ε-amino group of the peptide of the present invention to introduce the alkyne group. Thus, the "certain molecule" having the azide group can be covalently bound to the peptide of the present invention by the CuAAC reaction. The CuAAC reaction occurs as shown in Scheme 1 below.

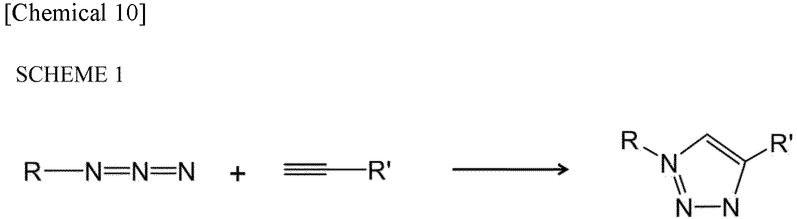

The CuAAC reaction produces 1,5-disubstituted-1,2,3-triazole. The reaction between alkyne and azide is highly selective, and natural biomolecules do not have the alkyne and azide groups. This reaction is rapid and pH-insensitive.

The alkyne group may be replaced by a cyclooctyne group. In some embodiments of the present disclosure, the peptide of the present invention includes an amino acid residue having an azide group at the N- or C-terminus. Thus, a "certain molecule" having a cyclooctyne group can be covalently bound to the N- or C-terminus of the peptide of the present invention by "strain-promoted azide-alkyne click chemistry (SPAAC) reaction." In another embodiment of the present disclosure, a linker having an azide group and an NHS ester group is reacted with the α- or ε-amino group of the peptide of the present invention to introduce the azide group. Thus, the "certain molecule" having the cyclooctyne group can be covalently bound to the peptide of the present invention by the SPAAC reaction. In another embodiment of the present disclosure, the peptide of the present invention includes an amino acid residue having a cyclooctyne group at the N- or C-terminus. Thus, a "certain molecule" having an azide group can be covalently bound to the N- or C-terminus of the peptide of the present invention by the SPAAC reaction. In another embodiment of the present disclosure, a linker having a cyclooctyne group and an NHS ester group is reacted with the α- or ε-amino group of the peptide of the present invention to introduce the cyclooctyne group. Thus, the "certain molecule" having the azide group can be covalently bound to the peptide of the present invention by the SPAAC reaction. The SPAAC reaction occurs as shown in Scheme 2 below.

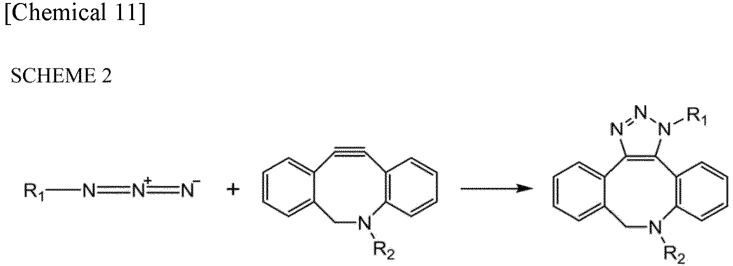

Alternatively, in some particular embodiments, a "certain molecule" having a tetrazine or cyclooctene group is bound to the corresponding cyclooctene or tetrazine group of a peptide or a linker by "inverse electron-demand Diels-Alder (iEDDA) reaction" as shown in Scheme 3 below.

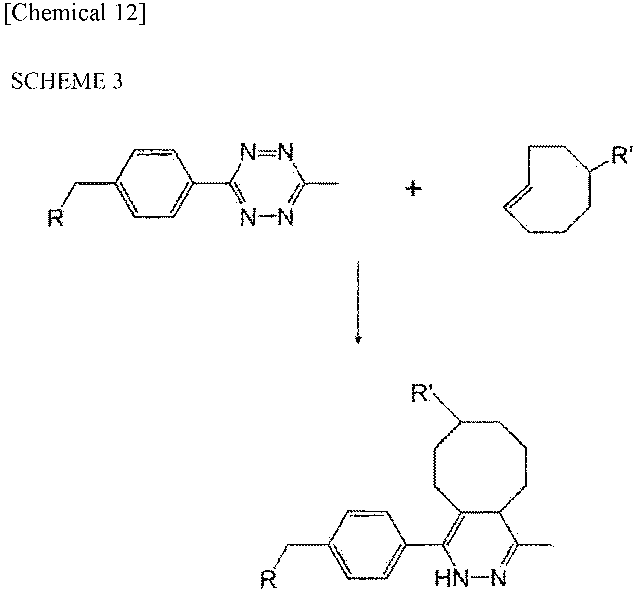

Examples of the amino acid residue having an azide group include L-azidohomoalanine (AHA), 4-azido-L-phenylalanine, 4-azido-D-phenylalanine, 3-azido-L-alanine, 3-azido-D-alanine, 4-azido-L-homoalanine, 4-azido-D-homoalanine, 5-azido-L-ornithine, 5-azido-d-ornithine, and 6-azido-L-lysine, and 6-azido-D-lysine. Examples of the amino acid residue having an alkyne group include L-homopropargylglycine (L-HPG), D-homopropargylglycine (D-HPG), and β-homopropargylglycine (β-HPG).

The strained alkyne group at the free end of the linker can be a cyclooctene group such as a trans-cyclooctene (TCO) group, or a cyclooctyne group such as a dibenzocyclooctyne (DBCO) group, a difluorocyclooctyne (DIFO) group, a bicyclononyne (BCN) group, and a dibenzocyclooctyne (DICO) group. Alternatively, the tetrazine group at the free end of the linker includes, but is not limited to, a 1,2,3,4-tetrazine group, a 1,2,3,5-tetrazine group, and a 1,2,4,5-tetrazine group, and derivatives thereof such as a 6-methyltetrazine group.

In a preferred embodiment of the present disclosure, the derivatives and modified forms of the peptide have an azide group, an alkyne group, or a tetrazine group at the N- or C-terminus or the amino acid side chain, either directly or via a linker. Examples of such derivatives are described below. For example, when DBCO-PEG4-NHS ester (D5922) or BCN-PEG4-NHS ester (BP-22851) is reacted with the epsilon amino group of lysine at the N-terminus of the peptide, peptides having DBCO or BCN added via a PEG linker (SEQ ID NOs: 49 and 50) are obtained.

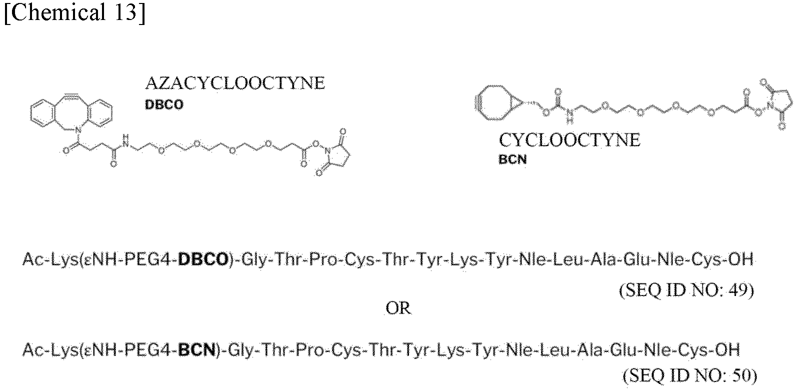

Alternatively, as shown in Scheme 4 below, when DBCO-PEG5-DBCO (BP-22450) is reacted with lysine at the N-terminus into which an azide group has been introduced by Fmoc-Lys(εN3)-OH, peptides with a DBCO group introduced into the amino acid side chain (SEQ ID NOs: 51 and 52) can be obtained.

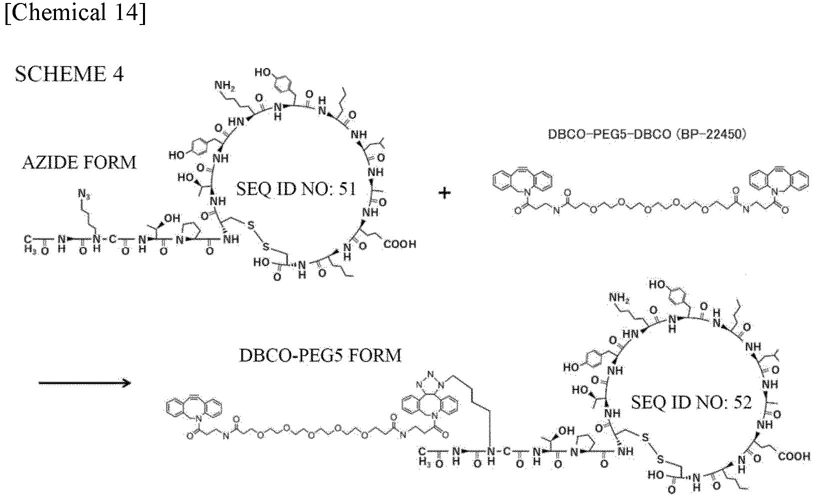

Further, in the following Scheme 5, N3-PEG4-NHS (N-succinimidyl 15-azido-4,7,10,13-tetraoxapentadecanoate) is reacted with the α-amino group of glycine at the N-terminus of the peptide of SEQ ID NO: 53 to synthesize a peptide having an azide group introduced therein (SEQ ID NO: 54), and a peptide having a DBCO group introduced therein at the N-terminus (SEQ ID NO: 55) can be obtained using DBCO-PEG5-DBCO.

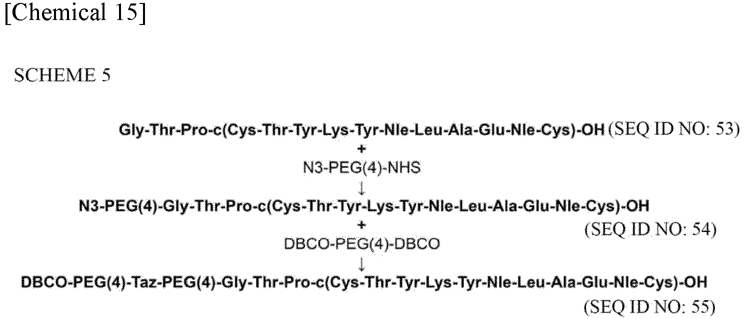

### (Peptide-Drug Conjugate)

The peptide of the present disclosure may be a conjugate. The conjugate is a conjugate of the peptide of the present disclosure, a linker bound to the peptide, and a "certain molecule" bound to the linker. The conjugate preferably allows at least the "certain molecule" to pass through the barrier tissue. The whole conjugate may be capable of passing through the barrier tissue.

Examples of the linker include, but are not limited to, those composed of about 1 to 15 amino acid residues, those composed of fatty acids, those composed of polyethylene glycol, and derivatives thereof. The linker may be one that dissociates or separates in a specific environment or condition or one that maintains a stable structure.

As used herein, a "certain molecule" can be any molecule that a person skilled in the art wishes to deliver to the brain. However, passing through the barrier tissue is achieved by RMT via LRP1 to which the molecule is bound, and thus, molecules too large to be transported by this mechanism are not preferred. Examples of the "certain molecule" include, but are not limited to, fluorescent dyes, low molecular weight compounds, peptides, peptoids, nucleic acids, sugar chains, lipids, antibodies, proteins, enzymes, liposomes, micelles, nanoparticles, carbon nanotubes, carbon nanohorns, carbon nanosheets, and those labeled with radioisotopes. In a preferred embodiment, the "certain molecule" is a drug, for example, an antibody, a nucleic acid, or a bioactive peptide.

The conjugate of the peptide of the present disclosure and the "certain molecule" may be a fusion or a graft. For example, the peptide of the present invention may be grafted to a loop region fused to the N- and/or C-terminus of an antibody or protein.

The fusion or the graft may be expressed as part of an ectodomain of a cell, a fungus, or a virus, or may be conjugated to the ectodomain of a cell, a fungus, or a virus.

The conjugate of the peptide of the present disclosure and the "certain molecule" may be formed via a "molecule having binding activity to the certain molecule." The peptide of the present disclosure and the "molecule having binding activity to the certain molecule" may be any of a conjugate, a fusion, and/or a graft. The binding between the "certain molecule" and the "molecule having binding activity to the certain molecule" may be reversible or irreversible.

The conjugate of the peptide of the present disclosure and the "certain molecule" may be a crystal, and the crystal is encompassed by the peptide of the present invention whether it is a monocrystal or a crystalline mixture. The crystal can be produced by crystallization by applying a crystallization method known per se.

The conjugate of the peptide of the present disclosure and the "certain molecule" may be a pharmaceutically acceptable co-crystal or co-crystal salt. The co-crystal or the co-crystal salt is a crystalline substance made of two or more unique solids at room temperature having different physical properties (e.g., structure, melting point, heat of fusion, hygroscopicity, solubility, and stability). The co-crystal or the co-crystal salt can be produced by a co-crystallization method known per se.

### (Effects of Cyclic Peptide)

As shown in Examples described later, Sequences 1 to 46, which are representative examples of the amino acid sequence group of the present embodiment, have the LRP1 binding activity. Sequence 2 moves from a blood well to brain well of an in vitro blood-brain barrier model, and has the ability to pass across the blood-brain barrier. The amino acid sequence group of the present embodiment has the amino acid sequences and three-dimensional structural characteristics similar to those of the representative examples. Thus, the amino acid sequence group is highly possible to have the LRP1 binding activity and the ability to pass through the blood-brain barrier. It is considered that the conjugate, the fusion, and the graft with the peptide of the present invention are also highly possible to have the LRP1 binding activity and the ability to pass the blood-brain barrier.

As shown in Examples described later, Sequences 1 to 46, which are representative examples of the amino acid sequence group of the present embodiment, have the LRP1 binding activity. This indicates the following. The bond between the Cα-carbon atoms of X⁵ and X¹⁵, the bond between the Cα-carbon atoms of X⁶ and X¹⁵, the bond between the Cα-carbon atoms of X⁵ and X¹⁶, or the bond between the Cα-carbon atoms of X⁶ and X¹⁶ accepts a wide variety of bonds, such as an amide bond, a disulfide bond, a thioether bond, a C=C bond, a C-C bond, a triazole-mediated bond, a dithiotetrafluorobenzene-mediated bond, and a dimethylphenylene group-mediated bond (the methylene group may be bound to the phenylene ring at any of the ortho, meta, and para positions).

The amino acid sequence of LRP1 cluster 4 is highly conserved among mammals, and Sequence 2, which is a representative example of the peptide of the present invention, shows the capability of passing through the blood-brain barrier in rat and monkey in vitro blood-brain barrier models. Thus, it is considered that the peptide group consisting of the amino acid sequences of the present embodiment and the conjugate of the peptide group and the "certain molecule" are highly possible to bind to LRP1 of mammals other than humans such as mice, rats, dogs, and monkeys, and show the capability of passing through the barrier tissue.

### (Method for Producing Cyclic Peptide)

The peptide of the present embodiment can be produced by a known method for producing a peptide, such as a chemical synthesis method including a liquid phase method, a solid phase method, or a hybrid method combining the liquid phase method and the solid phase method.

For the solid phase method, a commercially available automatic synthesizer can be used. For example, esterification is caused between a hydroxyl group of a resin having the hydroxyl group and a carboxy group of a first amino acid having the α-amino group protected by a protecting group such as an Fmoc group (generally a C-terminus amino acid of a target peptide). As an esterification catalyst, a known dehydration condensation agent such as 1-mesitylenesulfonyl-3-nitro-1,2,4-triazole (MSNT), dicyclohexylcarbodiimide (DCC), or diisopropylcarbodiimide (DIPCDI) can be used. Then, the protecting group at the α-amino group of the first amino acid is eliminated, a second amino acid in which all the functional groups other than the carboxy group of the main chain are protected is added, and the carboxy group is activated to bind the first and second amino acids. Further, the α-amino group of the second amino acid is deprotected, a third amino acid in which all the functional groups other than the carboxy group of the main chain are protected is added, and the carboxy group is activated to bind the second and third amino acids. This process is repeated to synthesize a peptide of a desired length. The linear peptide is cleaved from the resin and purified, and then the functional group for cyclizing the peptide is deprotected to cyclize the peptide according to a conventional method.

Examples of the resin for solid-phase synthesis include Merrifield resin, MBHA resin, Cl-Trt resin, SASRIN resin, Wang resin, Rink amide resin, HMFS resin, Amino-Merck (PEGA resin), HMPA-PEGA resin (Merck). These resins may be washed with a solvent (e.g., dimethylformamide (DMF), 2-propanol, or methylene chloride) before use. Examples of the protecting group for the α-amino group include a benzyloxycarbonyl (Cbz) group, a tert-butoxycarbonyl (Boc) group, a fluorenylmethoxycarbonyl (Fmoc) group, a benzyl group, an allyl group, and an allyloxycarbonyl (Alloc) group. The Cbz group can be deprotected with hydrofluoric acid or by hydrogenation, the Boc group can be deprotected with trifluoroacetic acid (TFA), and the Fmoc group can be deprotected by treatment with piperidine. The α-carboxy group can be protected with methyl ester, ethyl ester, benzyl ester, tert-butyl ester, or cyclohexyl ester. As for the other functional groups of amino acids, a hydroxy group such as serine and threonine can be protected with a benzyl group or a tert-butyl group, and a hydroxy group such as tyrosine can be protected with a 2-bromobenzyloxycarbonyl group or a tert-butyl group. The amino group of the side chain such as lysine and the carboxy group of glutamic acid or aspartic acid can be protected in the same manner as the α-amino group and the α-carboxy group.

The carboxy group can be activated using a condensing agent. Examples of the condensing agent include dicyclohexylcarbodiimide (DCC), diisopropylcarbodiimide (DIPCDI), 1-ethyl-3-(3-dimethylaminopropyl) carbodimide (EDC or WSC), (1H-benzotriazol-1-yloxy)tris(dimethylamino) phosphonium hexafluorophosphate (BOP), and 1-[bis(dimethylamino)methyl]-1H-benzotriazolium-3-oxide hexafluorophosphate (HBTU). The peptide chain can be cleaved from the resin by treatment with an acid such as TFA or hydrogen fluoride (HF).

In one embodiment, the peptide of the present disclosure may be cyclized. In the present specification, cyclization means that two or more amino acids separated by one or more amino acids are covalently bound directly or indirectly via a linker in one peptide to form one or more cyclic structures in the molecule. The cyclization can be achieved according to the methods described in Non-Patent Documents 8 and 9. The cyclization can be achieved by, for example, an amide bond between an amino group and a carboxy group, a disulfide bond between thiol groups, a thioether bond between a thiol group and a halogen group, a thioether bond between a thiol group and an allyl group by a thiol-ene reaction, a C=C bond between allyl groups by olefin metathesis (the C=C bond may be converted into a C-C bond by reduction), a triazole-mediated bond between an alkynyl group and an azide group by click reaction, and a thioether bond between a linker having a halogen group and two thiol groups, but is not limited thereto. The covalent bond for cyclization, which is direct or indirect via a linker, may be any of a main chain to main chain bond, a main chain to side chain bond, a side chain to main chain bond, or a side chain to side chain bond.

For cyclization of the peptide of the present disclosure, for example, the following bonds can be used: (1) a disulfide bond formed between thiol groups of cysteine, D-cysteine, homocysteine, and D-homocysteine each having the thiol group and independently defined as amino acid 1 and amino acid 2; (2) a thioether bond formed between amino acid 1 which is an amino acid (e.g., 3-chloroalanine) having a halogen atom (chloro, bromo, or iodo) as a nucleophilic group or a carboxylic acid (e.g., 3-chloropropionic acid) having a halogen atom (chloro, bromo, or iodo) and an amino acid 2 having a thiol group; (3) a thioether bond formed between amino acids 1 and 2 each having a thiol group via a linker having a halogen atom (chloro, bromo, or iodo) as a nucleophilic group (e.g., 1,1-diiodomethane, 1,1-dichloroacetone, 1,2-diiodoethane, 1,3-diiodopropane, 1,4-diiodobutane, α,α'-dibromo-o-xylene, α,α'-dibromo-m-xylene, α,α'-dibromo-p-xylene, or hexafluorobenzene); (4) a covalent bond formed by click reaction via triazole between amino acid 1 which is β-azidoalanine having an azide group and amino acid 2 which is 2-amino-5-hexynoic acid having an alkynyl group; (5) a thioether bond formed by thiol-ene reaction between amino acid 1 which is an amino acid having an allyl group (e.g., allylglycine, D-allylglycine, homoallylglycine, and D-homoallylglycine) or a carboxylic acid having an allyl group (e.g., 3-butenoic acid) and amino acid 2 having a thiol group; (6) a C=C bond formed by olefin metathesis between allyl groups of amino acids 1 and 2 which are each independently an amino acid having the allyl group or a carboxylic acid having the allyl group; (7) a C-C bond formed by reducing the C=C bond formed by olefin metathesis; (8) an amide bond formed between amino acid 1 having an amino group (e.g., 2,3-diaminopropanoic acid, 2,4-diaminobutanoic acid, ornithine, lysine, D-amino acids thereof, and β-alanine) and amino acid 2 having a carboxy group (e.g., aspartic acid, glutamic acid, and D-amino acids thereof) or a C-terminal carboxy group; and (9) an amide bond formed between the N-terminal amino group and amino acid having a carboxy group or a C-terminal carboxy group. Either amino acid 1 or amino acid 2 may be at the N-terminus.

### (Pharmaceutical, Diagnostic Agent, and Research Reagent Comprising Cyclic Peptide)

A pharmaceutical composition of the present disclosure comprises a peptide having the amino acid sequence described above as an active ingredient, and the peptide bound to LRP1 can be transported to central nervous system tissues by RMT via LRP1. The pharmaceutical composition may be administered in any form, and may be administered orally or parenterally. Examples of the parenteral administration include injection administration such as intramuscular injection, intravenous injection, and subcutaneous injection, transdermal administration, and transmucosal administration (nasal, transoral, transocular, pulmonary, vaginal, or transrectal administration). The peptide in the pharmaceutical composition can be modified in various ways in view of their constitutions to be metabolized and excreted. For example, alkyl chains, polyethylene glycols, or sugar chains can be added to the peptide to increase its retention time in the bloodstream and reduce its antigenicity. In addition, biodegradable polymeric compounds such as polylactic acid/glycol (PLGA), porous hydroxyapatite, liposomes, surface-modified liposomes, emulsions prepared with unsaturated fatty acids, nanoparticles, microparticles, nanospheres, etc. can be used as sustained-release base agents, and the peptides can be encapsulated in them. When the pharmaceutical composition is administered transdermally, a weak electric current can also be applied to the skin surface to penetrate the stratum corneum (iontophoresis method).

The pharmaceutical composition may be used as the active ingredient as it is, or may be formulated by adding a pharmaceutically acceptable carrier, an excipient, and an additive. Examples of the dosage form include liquids (e.g., injections), dispersants, suspensions, tablets, pills, powders, suppositories, sprays, fine granules, granules, capsules, syrups, lozenges, inhalants, ointments, eye drops, nasal drops, ear drops, and cataplasms. These formulations may be controlled release formulations (such as sustained-release microcapsules) such as fast release formulations or sustained-release formulations. The formulating can be carried out by a usual method using, for example, an excipient, a binder, a disintegrant, a lubricant, a dissolving agent, a solubilizing agent, a colorant, a flavoring agent, a stabilizing agent, an emulsifier, an absorption promoter, a surfactant, a pH adjusting agent, an antiseptic, and an antioxidant, as appropriate. Examples of ingredients used in formulating include, but are not limited to, purified water, saline solution, phosphate buffer solution, dextrose, pharmaceutically acceptable organic solvents such as glycerol and ethanol, as well as animal and vegetable oils, lactose, mannitol, glucose, sorbitol, crystal cellulose, hydroxypropyl cellulose, starch, corn starch, silicic anhydride, aluminum magnesium silicate, collagen, polyvinyl alcohol, polyvinylpyrrolidone, carboxyvinyl polymer, sodium carboxymethyl cellulose, sodium polyacrylate, sodium alginate, water-soluble dextran, sodium carboxymethyl starch, pectin, methylcellulose, ethylcellulose, xanthan gum, gum arabic, tragacanth, casein, agar, polyethylene glycol, diglycerin, glycerin, propylene glycol, vaseline, paraffin, octyldodecyl myristate, isopropyl myristate, higher alcohols, stearyl alcohol, stearic acid, and human serum albumin. When the peptide is difficult to be transmucosally absorbed, the following may be used as absorption promoters for improving absorption of poorly absorbable drugs: surfactants such as polyoxyethylene lauryl ethers, sodium lauryl sulfate, and saponin; bile salts such as glycocholic acid, deoxycholic acid, and taurocholic acid; chelating agents such as EDTA and salicylic acids; fatty acids such as caproic acid, capric acid, lauric acid, oleic acid, linoleic acid, and mixed micelles; an enamine derivative, an N-acylcollagen peptide, N-acylamino acid, cyclodextrins, chitosans, and a nitric oxide donor.

A pill or tablet can also be coated with a sugar coating, a gastrosoluble, or an enteric substance. The injection may comprise distilled water for injection, physiological saline, propylene glycol, polyethylene glycol, vegetable oils, alcohols, and the like. Humectants, emulsifiers, dispersants, stabilizing agents, dissolving agents, solubilizing agents, antiseptics, and the like may also be added. If necessary, additives such as normal antiseptics, antioxidants, colorants, sweetening agents, adsorbents, and humectants can be used in appropriate amounts.

The pharmaceutical composition of the present disclosure binds to LRP1 to carry a certain molecule to the central nervous system tissue, and is effective for treating or preventing various diseases targeted by the certain molecule. For example, if the certain molecule is an anticancer drug, the target may be brain tumors (e.g., pineal astrocytoma, pilocytic astrocytoma, diffuse astrocytoma, and anaplastic astrocytoma) or nerve sheath tumors. If the certain molecule is targeted to molecules related to central nervous system diseases, the target may be psychiatric disorders (e.g., schizophrenia, schizoaffective disorder, schizophreniform disorder, and delusional disorder), childhood mental disorders (e.g., attention deficit disorder, attention-deficit/hyperactivity disorder, conduct disorder, and autism), neurodegenerative disorder, neural stem cell disorder, neural progenitor disorder, ischemic disorder, neurotraumatic disorder, affective disorder, psychomotor disorder, sleep disorder (e.g., hypersomnia, circadian rhythm sleep disorder, insomnia, parasomnia, and sleep deprivation), mental disorders such as anxiety (e.g., acute stress disorder, generalized anxiety disorder, social anxiety disorder, panic disorder, posttraumatic stress disorder, agoraphobia, and obsessive-compulsive disorder), false psychiatric disorders (e.g., acute hallucinatory mania), impulse control disorders (e.g., compulsive gambling and intermittent explosive disorder), mood disorders (bipolar I disorder, bipolar II disorder, mania, and mixed affective state), major depression, chronic depression, seasonal depression, psychotic depression, seasonal depression, cognitive disorders (amnesia, senile dementia, HIV-associated dementia, Alzheimer's disease, Huntington's disease, dementia with Lewy body, vascular dementia, drug-related dementia, tardive dyskinesia, myoclonia, dystonia, delirium, Pick's disease, Creutzfeldt-Jakob disease, HIV disease, Gilles de la Tourette syndrome, epilepsy, muscle spasticity, and mild cognitive impairment), mental retardation (spasticity, Down syndrome, and fragile X syndrome), premenstrual syndrome (PMS), premenstrual dysphoric disorder (PDD), postpartum depression, neural damage disorders (e.g., ocular injury, ocular retinopathy or macular degeneration, tinnitus, hearing impairment, and cerebral edema), Parkinson's disease, parkinsonian disorders, migraine, epilepsy, Alzheimer's disease, brain injury, stroke, cerebrovascular diseases (e.g., cerebral arteriosclerosis, cerebral amyloid angiopathy, hereditary cerebral hemorrhage, and cerebral hypoxia-ischemia), drug addiction (e.g., narcotic addiction, alcoholism, amphetamine addiction, cocaine addiction, nicotine dependence, and drug withdrawal syndrome), and eating disorders (e.g., anorexia, bulimia, binge eating disorder, polyphagia, obesity, compulsive eating disorder, and pagophagia). However, the target is not limited to these diseases.

The pharmaceutical composition of the present disclosure may be administered in combination with other pharmaceuticals or therapeutic methods useful for the above diseases. For example, for treatment of malignant tumors, the composition may be combined with various kinds of chemotherapy, surgical treatment, and radiotherapy.

The dosage of the pharmaceutical composition of the present disclosure to mammals (e.g., humans, mice, rats, guinea pigs, rabbits, dogs, horses, monkeys, and pigs), particularly to humans, varies depending on the symptom, age, sex, body weight, and differences in sensitivities of a patient, administration methods, administration intervals, types of active ingredients, and types of preparations, and is not limited to a particular amount. For example, 30 µg to 1,000 mg, 100 µg to 500mg, or 100 µg to 100 mg of the composition can be administered once or in several doses.

### (Polynucleotide Encoding Peptide of the Present Disclosure)

**In** one embodiment, a polynucleotide encoding the peptide of the present disclosure is provided. This polynucleotide can be a polynucleotide encoding an amino acid sequence having only natural amino acids in the amino acid sequence of the peptide of the present disclosure, or a polynucleotide having a base sequence with a predetermined sequence identity to the polynucleotide. The degree of the base sequence identity may be about 70% or more, preferably about 80% or more, more preferably about 90% or more, still more preferably about 95% or more. The base sequence identity can be determined by a method known per se. For example, the base sequence identity (%) can be determined by the same method as the method for determining the amino acid sequence identity (%) described above.

**In** another embodiment, the polynucleotide can have a base sequence obtained by one or more modification, which is selected from substitution, addition, deletion, or insertion, of one or more nucleotides in the base sequence of the polynucleotide encoding the amino acid sequence having only the natural amino acids in the amino acid sequence of the peptide of the present disclosure. Any number of nucleotides can be modified as long as one or more nucleotides are modified. For example, 1 to about 50, preferably 1 to about 30, more preferably 1 to about 10, and even more preferably 1 to about 5 (e.g., 1 or 2) nucleotides can be modified.

### (Expression Vector)

An expression vector according to an embodiment of the present disclosure may comprise a polynucleotide encoding a target polypeptide to be expressed or a target polynucleotide to be expressed, and a promoter functionally linked to the polynucleotide. The "promoter functionally linked to the polynucleotide" means that the promoter is linked to the polynucleotide encoding the gene to allow the expression of the polynucleotide itself or the expression of the polypeptide encoded by the polynucleotide under the control of the promoter.

The expression vector of the present embodiment optionally has any backbone as long as a target substance can be produced in a predetermined cell. The backbone may be, for example, a plasmid vector or a viral vector. When the expression vector is used as a pharmaceutical, examples of vectors suitable for administration to mammals include viral vectors of adenovirus, retrovirus, adeno-associated virus, herpesvirus, vaccinia virus, poxvirus, poliovirus, Sindbis virus, and Sendai virus.

When a prokaryotic cell is used as a host cell, an expression vector that can use the prokaryotic cell as the host cell can be used. Such an expression vector may comprise elements such as a promoter-operator region, an initiation codon, a polynucleotide encoding the polypeptide of the present embodiment or its peptide portion, a stop codon, a terminator region, and a replication origin. The promoter-operator region for expressing the polypeptide of the invention in bacteria includes a promoter, an operator, and a Shine-Dalgarno (SD) sequence. As these elements, those known per se can be used.

When an eukaryotic cell is used as the host cell, an expression vector that can use the eukaryotic cell as the host cell can be used. In this case, any promoter can be used as long as the promotor can function in a eukaryote such as a mammal. When the expression of the polypeptide is intended, the promoter may be a viral promoter such as a SV40 early promoter, cytomegalovirus LTR, Rous sarcoma virus LTR, MoMuLV-LTR, and an adenovirus early promoter, and a promoter of mammalian constitutive protein genes such as a β-actin gene promoter, a PGK gene promoter, and a transferrin gene promoter. When the expression of the polynucleotide is intended, the promoter can be a pol III promoter (e.g., a tRNA promoter, a U6 promoter, and a H1 promoter).

The expression vector of the present invention may further comprise sites for transcription initiation and termination, and a ribosome binding site which may be required for translation in a transcription region, an origin of replication, and a selectable marker gene (e.g., ampicillin, tetracycline, kanamycin, spectinomycin, erythromycin, and chloramphenicol). The expression vector of the present invention can be prepared by a method known per se (see, e.g., Molecular Cloning described above).

### (Cell, Liposome, or Nanoparticle Presenting Peptide of the Present Disclosure)

In another aspect of the present disclosure, a cell, liposome, or nanoparticle presenting the peptide of the present disclosure is provided. The cell can be produced by isolating a desired cell from a living body and conjugating the peptide of the present disclosure to the cell in vitro, or introducing a vector expressing the peptide of the present disclosure into the cell and allowing the peptide to be presented on the cell surface. The liposome or nanoparticle can be produced by conjugating the peptide of the present disclosure or preparing a conjugate of the peptide of the present disclosure and a fatty chain and inserting the conjugate into a lipid membrane via the fatty chain to allow the peptide to be presented on the surface of the liposome or nanoparticle.

The following examples are merely illustrative, and are intended only to describe the present invention in detail together with the above-described embodiments, and are not intended to limit the present invention. A person skilled in the art can change the present invention to various ways without departing from the meaning of the present invention, and such changes are also included in the scope of the present invention.

### [Examples]

The abbreviations used in the present specification have the following meanings.
BSA: Bovine serum albumin
RP-HPLC: Reverse-phase high performance liquid chromatography
HRP: Horse radish peroxidase
SA: Streptavidin
D-PBS: Dulbecco's phosphate buffered saline
ELISA: Enzyme-linked immunosorbent assay
Ac: Acetyl
Cys: L-cysteine
Gly: Glycine
Ala: L-alanine
Ser: L-serine
Thr: L-threonine
Pro: L-proline
cHyp: cis-4-hydroxy-L-proline
tHyp: trans-4-hydroxy-L-proline
Tyr: L-tyrosine
Trp: L-tryptophan
Phe: L-phenylalanine
4fF: 4-fluoro-L-phenylalanine
4cF: 4-chloro-L-phenylalanine
His: L-histidine
Lys: L-lysine
Orn: L-ornithine
Arg: L-arginine
Val: L-valine
Leu: L-leucine
Ile: L-isoleucine
Nle: L-norleucine
Ahep: (S)-2-aminoheptanonic acid
Aoc: (S)-2-aminooctanonic acid
Aib: 2-aminoisobutyric acid
Asp: L-aspartic acid
Glu: L-glutamic acid
5-FAM: 5-carboxy fluorescein
DX: D-amino acid X
c(XY): Cyclization between amino acid X to amino acid Y

### (Peptide Synthesis)

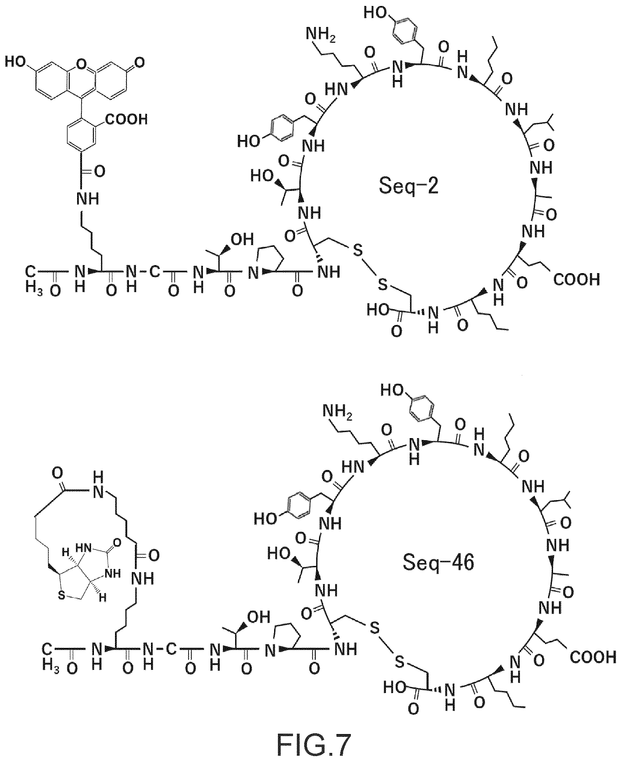

The chemical synthesis of all peptides used in the present example was outsourced to SCRUM Inc. (Tokyo, Japan), a standard solid phase synthesis method using a 9-fluorenylmethoxycarbonyl group (Fmoc group) as a protecting group of an α-amino group was performed by an automatic synthesizer Syro II (manufactured by Biotage). The C-terminus side-chain protected amino acid-resin was placed in the synthesis column, and the column was set in the synthesizer. Subsequently, 1-[bis(dimethylamino)methylene]-1H-1,2,3-triazolo[4,5-b]pyridinium 3-oxidehexafluorophosphate (HATU)/diisopropylethylamine (DIEA) was added to the next amino acid protected with the Fmoc group to activate the next amino acid, and the mixture was placed in a column and allowed to react. After the reaction was completed, the resultant was washed and the Fmoc group was deprotected using 20% piperidine. By repeating this step, the peptide chain was extended, the Fmoc group of the final amino acid was deprotected, and then the peptide resin was removed from the synthesizer. To the peptide resin, 30% hexafluoro-2-propanol (HFIP)/dichloromethane (DCM) was added to cleave a side chain-protected linear peptide from the resin, and the side chain-protected peptide was recovered by ether precipitation. The side chain-protected peptide was purified by RP-HPLC using a SunFire C18 column (10 mm × 150 mm) (manufactured by Waters), and then lyophilized. The cyclization of the peptide was performed with reference to the methods described in Non-Patent Documents 8 and 9. Tables 1 and 2 show the theoretical molecular weights, measured molecular weights, purity, types of cyclization, and amino acid sequences of the peptides synthesized in this example. FIG. 7 shows the structural formulae of Sequences 2 and 46. In Tables 1 and 2, amino acid residues without D indicate L amino acid residues.

**[Table 1]**

| Name | Calcd MW | Obsvd MW | Mode | Purity (%) | Cyclization | Sequence |
|---|---|---|---|---|---|---|
| SEQUENCE 1 | 2101.3 | 2101.906 | NEG | 73 | None | Ac-Lys(5FAM)-Gly-Thr-Pro-Cys-Thr-Tyr-Lys-Tyr-Nle-Leu-Ala-Glu-Nle-Cys)-OH (SEQ ID NO: 1) |
| SEQUENCE 2 | 2099.3 | 2099.4 | POS | 95 | S-S(5-15) | Ac-Lys(5FAM)-Gly-Thr-Pro-c(Cys-Thr-Tyr-Lys-Tyr-Nle-Leu-Ala-Glu-Nle-Cys)-OH (SEQ ID NO: 2) |
| SEQUENCE 3 | 2113.3 | 2113.947 | POS | 100 | S-C-S(5-15) | Ac-Lys(5FAM)-Gly-Thr-Pro-c(Cys-Thr-Tyr-Lys-Tyr-Nle-Leu-Ala-Glu-Nle-Cys)-OH (SEQ ID NO: 3) |
| SEQUENCE 4 | 2141.4 | 2141.838 | POS | 90 | S-C3-S(5-15) | Ac-Lys(5FAM)-Gly-Thr-Pro-c(Cys-Thr-Tyr-Lys-Tyr-Nle-Leu-Ala-Glu-Nle-Cys)-OH (SEQ ID NO: 4) |
| SEQUENCE 5 | 2101.3 | 2102.233 | POS | 99 | None | Ac-Lys(5FAM)-Gly-Thr-Pro-^{D}Cys-Thr-Tyr-Lys-Tyr-Nle-Leu-Ala-Glu-Nle-Cys-OH (SEQ ID NO: 5) |
| SEQUENCE 6 | 2099.3 | 2099.962 | POS | 100 | S-S(5-15) | Ac-Lys(5FAM)-Gly-Thr-Pro-c(^{D}Cys-Thr-Tyr-Lys-Tyr-Nle-Leu-Ala-Glu-Nle-Cys)-OH (SEQ ID NO: 6) |
| SEQUENCE 7 | 2113.3 | 2113.69 | POS | 98 | S-C-S(5-15) | Ac-Lys(5FAM)-Gly-Thr-Pro-c(^{D}Cys-Thr-Tyr-Lys-Tyr-Nle-Leu-Ala-Glu-Nle-Cys)-OH (SEQ ID NO: 7) |
| SEQUENCE 8 | 2141.4 | 2142.991 | POS | 77 | S-C3-S(5-15) | Ac-Lys(5FAM)-Gly-Thr-Pro-c(^{D}Cys-Thr-Tyr-Lys-Tyr-Nle-Leu-Ala-Glu-Nle-Cys)-OH (SEQ ID NO: 8) |
| SEQUENCE 9 | 2101.3 | 2102.504 | POS | 99 | None | Ac-Lys(5FAM)-Gly-Thr-Pro-Cys-Thr-Tyr-Lys-Tyr-Nle-Leu-Ala-Glu-Nle-^{D}Cys-OH (SEQ ID NO: 9) |
| SEQUENCE 10 | 2099.3 | 2099.154 | POS | 100 | S-S(5-15) | Ac-Lys(5FAM)-Gly-Thr-Pro-c(Cys-Thr-Tyr-Lys-Tyr-Nle-Leu-Ala-Glu-Nle-^{D}Cys)-OH (SEQ ID NO: 10) |
| SEQUENCE 11 | 2113.3 | 2113.114 | POS | 100 | S-C-S(5-15) | Ac-Lys(5FAM)-Gly-Thr-Pro-c(Cys-Thr-Tyr-Lys-Tyr-Nle-Leu-Ala-Glu-Nle-^{D}Cys)-OH (SEQ ID NO: 11) |
| SEQUENCE 12 | 2141.4 | 2141.921 | POS | 99 | S-C3-S(5-15) | Ac-Lys(5FAM)-Gly-Thr-Pro-c(Cys-Thr-Tyr-Lys-Tyr-Nle-Leu-Ala-Glu-Nle-^{D}Cys)-OH (SEQ ID NO: 12) |
| SEQUENCE 13 | 2101.3 | 2101.122 | POS | 99 | None | Ac-Lys(5FAM)-Gly-Thr-Pro-^{D}Cys-Thr-Tyr-Lys-Tyr-Nle-Leu-Ala-Glu-Nle-^{D}Cys-OH (SEQ ID NO: 13) |
| SEQUENCE 14 | 2099.3 | 2099.328 | POS | 96 | S-S(5-15) | Ac-Lys(5FAM)-Gly- Thr-Pro-c(^{D}Cys-Thr-Tyr-Lys-Tyr-Nle-Leu-Ala-Glu-Nle-^{D}Cys)-OH (SEQ ID NO: 14) |
| SEQUENCE 15 | 2115.3 | 2114.823 | POS | 98 | S-C-S(5-15) | Ac-Lys(5FAM)-Gly-Thr-Pro-c(^{D}Cys-Thr-Tyr-Lys-Tyr-Nle-Leu-Ala-Glu-Nle-^{D}Cys)-OH (SEQ ID NO: 15) |
| SEQUENCE 16 | 2141.4 | 2141.838 | POS | 98 | S-C3-S(5-15) | Ac-lys(5FAM)-Gly-Thr-Pro-c(^{D}Cys-Thr-Tyr-Lys-Thr-Nle-Leu-Ala-Glu-Nle-^{D}Cys)-OH (SEQ ID NO: 16) |
| SEQUENCE 17 | 2085.3 | 2085.897 | POS | 95 | S-S(5-15) | Ac-Lys(5FAM)-Gly-Thr-Pro-c(Cys-Ser-Tyr-Lys-Tyr-Nle-Leu-Ala-Glu-Nle-Cys)-OH (SEQ ID NO: 17) |
| SEQUENCE 18 | 2111.3 | 2111.941 | POS | 99 | S-S(5-15) | Ac-Lys(5FAM)-Gly-Thr-Pro-c(Cys-cHyp-Tyr-Lys-Tyr-Nle-Leu-Ala-Glu-Nle-Cys)-OH (SEQ ID NO: 18) |
| SEQUENCE 19 | 2111.3 | 2111.103 | NEG | 95 | S-S(5-15) | Ac-Lys(5FAM)-Gly-Thr-Pro-c(Cys-tHyp-Tyr-Lys-Tyr-Nle-Leu-Ala-Glu-Nle-Cys)-OH (SEQ ID NO: 19) |
| SEQUENCE 20 | 2083.3 | 2086.899 | NEG | 97 | S-S(5-15) | Ac-Lys(5FAM)-Gly-Thr-Pro-c(Cys-Thr-Phe-Lys-Tyr-Nle-Leu-Ala-Glu-Nle-Cys)-OH (SEQ ID NO: 20) |
| SEQUENCE 21 | 2122.3 | 2122.918 | NEG | 95 | S-S(5-15) | Ac-Lys(5FAM)-Gly-Thr-Pro-c(Cys-Thr-Trp-Lys-Tyr-Nle-Leu-Ala-Glu-Nle-Cys)-OH (SEQ ID NO: 21) |
| SEQUENCE 22 | 2073.3 | 2074.369 | POS | 97 | S-S(5-15) | Ac-Lys(5FAM)-Gly-Thr-Pro-c(Cys-Thr-His-Lys-Tyr-Nle-Leu-Ala-Glu-Nle-Cys)-OH (SEQ ID NO: 22) |
| SEQUENCE 23 | 2085.3 | 2085.859 | POS | 90 | S-S(5-15) | Ac-Lys(5FAM)-Gly-Thr-Pro-c(Cys-Thr-Tyr-Orn-Tyr-Nle-Leu-Ala-Glu-Nle-Cys)-OH (SEQ ID NO: 23) |
| SEQUENCE 24 | 2127.3 | 2127.357 | POS | 98 | S-S(5-15) | Ac-Lys(5FAM)-Gly-Thr-Pro-c(Cys-Thr-Tyr-Arg-Tyr-Nle-Leu-Ala-Glu-Nle-Cys)-OH (SEQ ID NO: 24) |

**[Table 2]**

| Name | Calcd MW | Obsvd MW | Mode | Purity (%) | Cyclization | Sequence |
|---|---|---|---|---|---|---|
| SEQUENCE 25 | 2049.3 | 2050.56 | NEG | 96 | S-S(5-15) | Ac-Lys(5FAM)-Gly-Thr-Pro-c(Cys-Thr-Tyr-Lys-Nle-Nle-Leu-Ala-Glu-Nle-Cys)-OH (SEQ ID NO: 25) |
| SEQUENCE 26 | 2135.3 | 2134.939 | NEG | 96 | S-S(5-15) | Ac-Lys(5FAM)-Gly-Thr-Pro-c(Cys-Thr-Tyr-Lys-Arg-Arg-Leu-Ala-Glu-Nle-Cys)-OH (SEQ ID NO: 26) |
| SEQUENCE 27 | 2142.3 | 2142.915 | POS | 95 | S-S(5-15) | Ac-Lys(5FAM)-Gly-Thr-Pro-c(Cys-Thr-Tyr-Lys-Tyr-Arg-Leu-Ala-Glu-Nle-Cys)-OH (SEQ ID NO: 27) |
| SEQUENCE 28 | 2085.3 | 2084.384 | NEG | 96 | S-S(5-15) | Ac-Lys(5FAM)-Gly- Thr-Pro-c(Cys-Thr-Tyr-Lys-Tyr-Nle-Val-Ala-Glu-Nle-Cys)-OH (SEQ ID NO: 28) |
| SEQUENCE 29 | 2099.3 | 2099.841 | POS | 95 | S-S(5-15) | Ac-Lys(5FAM)-Gly-Thr-Pro-c(Cys-Thr-Tyr-Lys-Tyr-Nle-Ile-Ala-Glu-Nle-Cys)-OH (SEQ ID NO: 29) |
| SEQUENCE 30 | 2099.3 | 2099.247 | POS | 95 | S-S(5-15) | Ac-Lys(5FAM)-Gly-Thr-Pro-c(Cys-Thr-Tyr-Lys-Tyr-Nle-Nle-Ala-Glu-Nle-Cys)-OH (SEQ ID NO: 30) |
| SEQUENCE 31 | 2113.3 | 2113.929 | NEG | 100 | S-S(5-15) | Ac-Lys(5FAM)-Gly-Thr-Pro-c(Cys-Thr-Tyr-Lys-Tyr-Nle-Ahep-Ala-Glu-Nle-Cys)-OH (SEQ ID NO: 31) |
| SEQUENCE 32 | 2127.4 | 2127.928 | POS | 96 | S-S(5-15) | Ac-Lys(5FAM)-Gly-Thr-Pro-c(Cys-Thr-Tyr-Lys-Tyr-Nle-Aoc-Ala-Glu-Nle-Cys)-OH (SEQ ID NO: 32) |
| SEQUENCE 33 | 2099.3 | 2099.997 | NEG | 91 | S-S(5-15) | Ac-Lys(5FAM)-Gly-Thr-Pro-c(Cys-Thr-Tyr-Lys-Tyr-Nle-Leu-DAla-Glu-Nle-Cys)-OH (SEQ ID NO: 33) |
| SEQUENCE 34 | 2113.3 | 2113.833 | NEG | 96 | S-S(5-15) | Ac-Lys(5FAM)-Gly-Thr-Pro-c(Cys-Thr-Tyr-Lys-Tyr-Nle-Leu-Aib-Glu-Nle-Cys)-OH (SEQ ID NO: 34) |
| SEQUENCE 35 | 2084.3 | 2085.861 | NEG | 99 | S-S(5-15) | Ac-Lys(5FAM)-Gly-Thr-Pro-c(Cys-Thr-Tyr-Lys-Tyr-Nle-Leu-Ala-Asp-Nle-Cys)-OH (SEQ ID NO: 35) |
| SEQUENCE 36 | 2041.3 | 2042.942 | POS | 95 | S-S(5-15) | Ac-Lys(5FAM)-Gly-Thr-Pro-c(Cys-Thr-Tyr-Lys-Tyr-Nle-Leu-Ala-Ala-Nle-Cys)-OH (SEQ ID NO: 36) |
| SEQUENCE 37 | 2027.2 | 2027.255 | NEG | 95 | S-S(5-15) | Ac-Lys(5FAM)-Gly-Thr-Pro-c(Cys-Thr-Tyr-Lys-Tyr-Nle-Leu-Ala-Gly-Nle-Cys)-OH (SEQ ID NO: 37) |
| SEQUENCE 38 | 2085.3 | 2085.807 | POS | 95 | S-S(5-15) | Ac-Lys(5FAM)-Gly-Thr-Pro-c(Cys-Thr-Tyr-Lys-Tyr-Nle-Leu-Ala-Glu-Val-Cys)-OH (SEQ ID NO: 38) |
| SEQUENCE 39 | 2099.3 | 2099.413 | NEG | 88 | S-S(5-15) | Ac-Lys(5FAM)-Gly-Thr-Pro-c(Cys-Thr-Tyr-Lys-Tyr-Nle-Leu-Ala-Glu-Leu-Cys)-OH (SEQ ID NO: 39) |
| SEQUENCE 40 | 2099.3 | 2099.813 | POS | 96 | S-S(5-15) | Ac-Lys(5FAM)-Gly-Thr-Pro-c(Cys-Thr-Tyr-Lys-Tyr-Nle-Leu-Ala-Glu-Ile-Cys)-OH (SEQ ID NO: 40) |
| SEQUENCE 41 | 2113.3 | 2113.904 | POS | 97 | S-S(5-15) | Ac-Lys(5FAM)-Gly-Thr-Pro-c(Cys-Thr-Tyr-Lys-Tyr-Nle-Leu-Ala-Glu-Ahep-Cys)-OH (SEQ ID NO: 41) |
| SEQUENCE 42 | 2127.3 | 2127.864 | POS | 95 | S-S(5-15) | Ac-Lys(5FAM)-Gly-Thr-Pro-c(Cys-Thr-Tyr-Lys-Tyr-Nle-Leu-Ala-Glu-Aoc-Cys)-OH (SEQ ID NO: 42) |
| SEQUENCE 43 | 2196.4 | 2196.703 | NEG | 95 | S-S(5-16) | Ac-Lys(5FAM)-Gly-Thr-Pro-c(Cys-Thr-Tyr-Lys-Tyr-Nle-Leu-Ala-Glu-Nle-Pro-Cys)-OH (SEQ ID NO: 43) |
| SEQUENCE 44 | 2196.4 | 2196.878 | NEG | 96 | S-S(6-16) | Ac-Lys(5FAM)-Gly-Thr-Pro-Thr-c(Cys-Tyr-Lys-Tyr-Nle-Leu-Ala-Glu-Nle-Pro-Cys)-OH (SEQ ID NO: 44) |
| SEQUENCE 45 | 1970.3 | 1971.794 | NEG | 95 | None | Ac-Lys(Biotin)-Gly-Thr-Pro-Cys-Thr-Tyr-Lys-Tyr-Nle-Leu-Ala-Glu-Nle-Cys-OH (SEQ ID NO: 45) |
| SEQUENCE 46 | 1968.3 | 1969.916 | NEG | 96 | S-S | Ac-Lys(Biotin)-Gly-Thr-Pro-c(Cys-Thr-Tyr-Lys-Tyr-Nle-Leu-Ala-Glu-Nle-Cys)-OH (SEQ ID NO: 46) |
| SEQUENCE 47 | 2845.0 | 2846.358 | NEG | 99 | None | Lys(5FAM)-Gly-Thr-Phe-Phe-Tyr-Gly-Gly-Ser-Arg-Gly-Lys-Arg-Asn-Asn-Phe-Lys-The-Glu-Glu-Tyr-OH (SEQ ID NO: 47) |
| SEQUENCE 48 | 3069.7 | 3070.886 | POS | 96 | None | Ac-Lys(5FAM)-Gly-Thr-Trp-Pro-Lys-His-Phe-Asp-Lys-His-Thr-Phe-Tyr-Ser-Ile-Leu-Lys-Leu-Gly-Lys-His-OH (SEQ ID NO: 48) |

### (Construction of Binding Test by ELISA)

To evaluate the binding activity of the peptides to LRP1, an ELISA-based binding assay was constructed. The ELISA method will be briefly described below. First, a goat antihuman IgG-Fcγ polyclonal antibody (Catalog No. 109-0005-008, manufactured by Jackson Immuno Research) was added to a 96-well Maxisorp plate (Catalog No. 439454, manufactured by Nunc) at 1 µg/50 µL/well using D-PBS, followed by coating overnight at 4°C. Then, 0.5% BSA/D-PBS was further added at 300 µL/well, and blocking was carried out at room temperature for 30 minutes. After washing the plate with 0.1% Tween 20/D-PBS, LRP1 recombinant proteins, namely, LRP1 cluster 2-Fc chimeric proteins (Catalog No. 2368-L2-050, manufactured by R & D Systems), LRP1 cluster 3-Fc chimeric proteins (Catalog No. 48248-L3-050, manufactured by R & D Systems), or LRP1 cluster 4-Fc chimeric proteins (Catalog No. 5395-L4-050, manufactured by R & D Systems), were added at 100 ng/50 µL/well using 0.5% BSA/D-PBS, and allowed to react with the coated antibodies at room temperature for 30 minutes. After washing the plate with 0.1% Tween 20/PBS, Sequences 45 and 46, which were biotinylated peptides, were prepared at an arbitrary concentration using 0.5% BSA/D-PBS, and added at 50µL/well. After causing a reaction at room temperature for 30 minutes, the plate was washed with 0.1% Tween 20/D-PBS. Sequences 45 and 46 bound to the LRP1 recombinant proteins captured on the plate were detected with SA-HRP (Catalog No. ab7403, manufactured by Abeam). For quantification of HRP, TMB ELISA Substrate Solution (Catalog No. 34028, manufactured by Thermo Fisher) was used to measure absorbance at 450 nm.

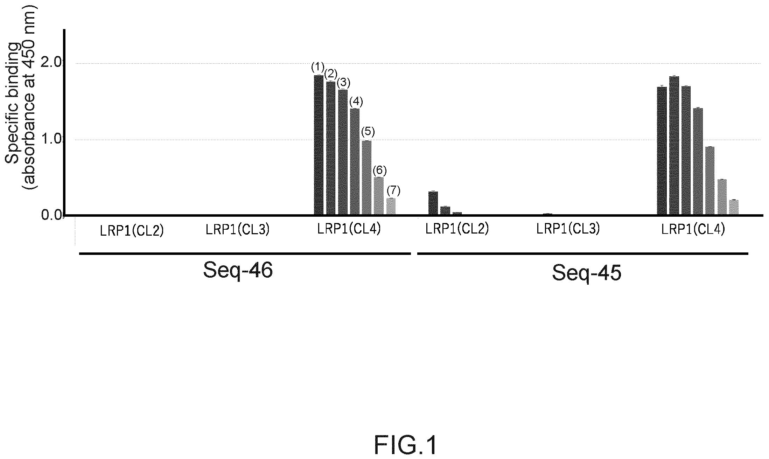

FIG. 1 shows the selective and peptide concentration-dependent binding of Sequences 45 and 46 used to test the binding activity to LRP1 cluster 4-Fc chimeric proteins (n = 4, ± SEM). The specific binding activity was estimated by subtracting the absorbance obtained by adding the biotinylated peptide to the wells in which no LRP1 recombinant proteins were captured from the absorbance obtained by adding the biotinylated peptide to the wells in which the LRP1 recombinant proteins were captured. As a result, both of Sequences 45 and 46 bound to the LRP1 cluster 4-Fc chimeric proteins selectively and in a peptide concentration-dependent manner. The sequences showed little or no binding to the LRP1 cluster 2-Fc chimeric proteins and the LRP1 cluster 3-Fc chimeric proteins. The binding EC50 values of Sequences 45 and 46 were calculated as 10.5 nM and 11.0 nM, respectively, indicating that the sequences had similar binding activities whether the cyclic structure is present or not.

### (Construction of Competitive Binding Assay by ELISA)

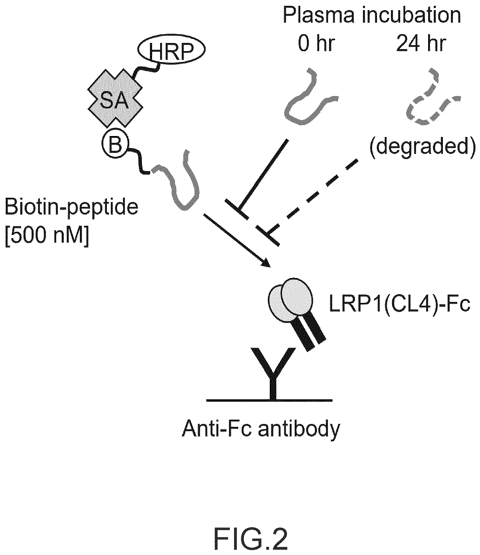

For comparison between the peptide of Sequence 1 with amino acid substitutions and the peptide of Sequence 2 in terms of the LRP1 binding activity and the resistance to protease degradation, a competitive binding assay by ELISA shown in FIG. 2 was constructed. Immediately (zero hour) or 24 hours after the mixing of the peptide of Sequence 2 or the amino acid-substituted peptide with mouse plasma at 37°C, the mixture was diluted to sufficiently lower the plasma concentration to any desired final concentration, and mixed with Sequence 46 (500 nM). This mixture was subjected to the competitive binding assay described above. The binding of Sequence 46 to the LRP1 cluster 4-Fc chimera proteins competes with the binding of Sequence 2 or the amino acid-substituted peptide to the LRP1 cluster 4-Fc chimera proteins, and thus is inhibited depending on the concentration of Sequence 2 or the amino acid-substituted peptide. That is, the binding activity of Sequence 2 or the amino acid-substituted peptide to the LRP1 cluster 4-Fc chimera proteins is detected as competitive inhibitory activity against the binding of Sequence 46. Regarding the binding value of Sequence 46 to the wells in which the LRP1 cluster 4-Fc chimeric proteins were not captured as 100% inhibitory activity and the binding value of Sequence 46 to the wells in which Sequence 2 or the amino acid-substituted peptide was not added as 0% inhibitory activity, the inhibitory activity values of Sequence 2 and the amino acid-substituted peptide were calculated in %.

### (Evaluation of Binding Activity of Amino Acid-Substituted Peptide to LRP1)

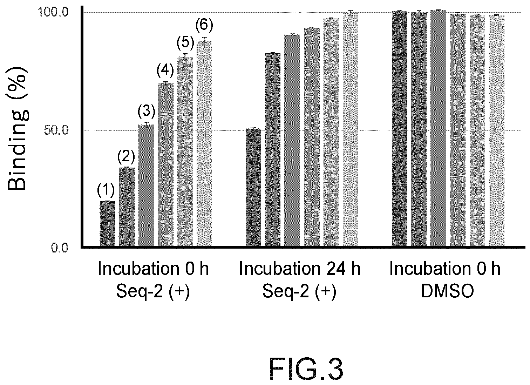

FIG. 3 shows the results of the competition binding test for the LRP1 cluster 4-Fc chimeric proteins, n = 4 (± SEM). Sequence 2 incubated with plasma for zero hour inhibited the binding of Sequence 46 in a concentration-dependent manner. The binding of Sequence 46 (500nM) in wells with 500 nM Sequence 2 averaged 52.4%, indicating that Sequences 2 and 46 competed 1: 1 for binding to the LRP1 Cluster 4. Sequence 2 incubated with plasma for 24 hours also inhibited the binding of Sequence 46 in a concentration-dependent manner. However, the binding of Sequence 46 (500 nM) in wells loaded with 2,000 nM equivalent Sequence 2 averaged 50.6%. That is, the inhibitory activity exhibited was as low as 500 nM although the amount corresponding to 2,000 nM was added to the wells. This indicates that approximately 75% of Sequence 2 was degraded during the 24-hour incubation with the mouse plasma. When DMSO was tested in the same manner immediately after the mixing with the mouse plasma, the binding of Sequence 46 (500 nM) was not inhibited at all, indicating that the plasma contaminating the binding system did not inhibit the system.

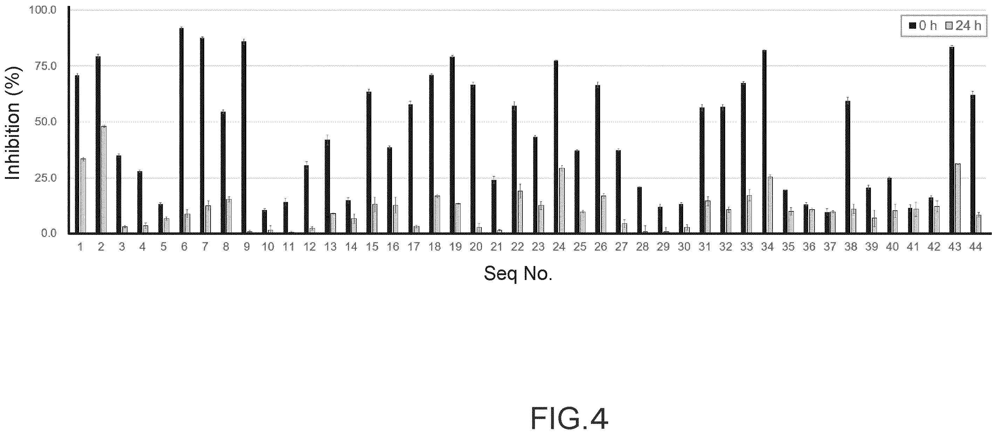

Immediately or 24 hours after Sequences 1 to 44 were mixed with mouse plasma at 37°C, the mixtures were diluted to sufficiently lower the plasma concentration to a final concentration of 2,000 nM, and mixed with Sequence 46 (500 nM). The obtained mixtures were subjected to the above-described competitive binding assay. FIG. 4 shows the results. The inhibitory activities of Sequences 1 and 2 were estimated to be 70.7% and 79.4%, respectively. The inhibitory activities of Sequences 6, 7, 9, 19, 24, 34, and 43 were 91.9%, 87.6%, 85.8%, 79.1%, 77.4%, 82.0%, and 83.5%, respectively, which were equivalent to or higher than those of Sequences 1 and 2. Other sequences tended to have diminished inhibitory activities.
As with Sequence 2, many of the amino acid-substituted peptides uniformly showed reduced inhibitory activity 24 hours after mixing with the mouse plasma, compared to the samples subjected to the competition test immediately after mixing with the mouse plasma. This indicates that the peptides were degraded in the mouse plasma.

### (Evaluation by In Vitro Blood-Brain Barrier Model)

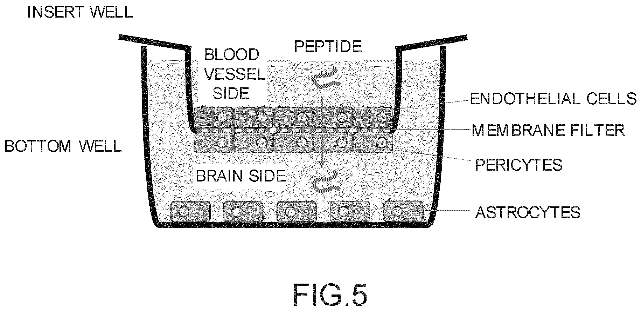

FIG. 5 is a schematic view of an in vitro blood-brain barrier model for assessing the permeability through the blood-brain barrier. Rat BBB Kit (catalog No. RBT-24H, manufactured by Pharmacocel Inc.) and monkey BBB Kit (catalog No. MBT-24H, manufactured by Pharmacocel Inc.) were used for the test. These kits retain the characteristics of BBB in vivo (whether various receptors and transporters are expressed and the ability to form tight junctions), and are widely used as an evaluation system for estimating drug transport to the brain (Non-Patent Documents 6 and 7). Using an insert well as a blood vessel side and a bottom well as a brain side, how much of the peptide added to the insert well penetrated a three-layer bottom surface made of endothelial cells/membrane filter/pericytes was evaluated. A transepithelial electrical resistance value (TEER) indicating the BBB function was measured before and after the test to confirm that the addition of the peptide did not affect the BBB function.

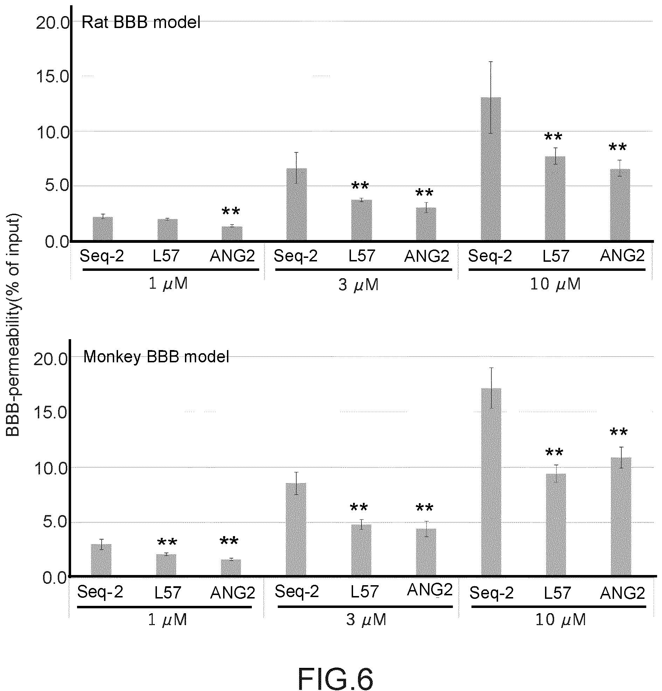

FIG. 6 shows the evaluation results of the BBB permeability of Sequence 2, in comparison with ANG2 (Non-Patent Document 4) and L57 (Non-Patent Document 5), which have been reported to have the LRP1 binding activity and the BBB permeability. To the insert wells, 5-FAM-labeled ANG (Sequence 47), 5-FAM-labeled L57 (Sequence 48), and Sequence 2 were added to final concentrations of 10 µM, 3 µM, and 1 µM, respectively, and incubated at 37°C for 24 hours. Then, the culture supernatants in the bottom wells were recovered. Each 5-FAM-labeled peptide was serially diluted with a medium to any concentration to prepare a calibration curve, and the concentration of the 5-FAM-labeled peptide contained in each recovered culture supernatant was calculated. The amount (amount by mole) of the peptide transferred to the bottom well (brain side) was calculated as a percentage, regarding the amount (amount by mole) of the peptide added to the insert well (blood vessel side) as 100%. Sequence 2 showed the significantly higher BBB permeability than 5-FAM-labeled ANG and L57 at any concentration whether the rat BBB shown in the upper graph or the monkey BBB shown in the lower graph was used (n = 4, ± SD, *p<0.01, Dunnett's test on Sequence 2). After 24 hour incubation, all wells showed transepithelial electric resistance (TEER) values of 150 Ω × cm² or more, indicating that the BBB function was maintained. These results indicate that the peptides of the present invention can pass through the barrier tissue, particularly the blood-brain barrier. It was also confirmed that Sequence 2, a representative example of the peptide of the present invention, showed the greater BBB permeability than the existing ANG2 or L57.

### (Synthesis and Evaluation of Peptide Modified Form DBCO-KS-487)

### <Synthesis Method>

The synthesis was outsourced to SCRUM Inc. (Tokyo, Japan). The following linear peptide precursor was first synthesized by standard solid-phase synthesis using 9-fluoromethoxycarbonyl group (Fmoc) as the α-amino protecting group on an automated synthesizer Syru II (manufactured by Biotage).
NH₂-Gly-Thr(tBu)-Pro-Cys(Trt)-Thr(tBu)-Tyr(tBu)-Lys(Boc)-Tyr(tBu)-Nle-Leu-Ala-Glu(OtBu)-Nle-Cys(Trt)-Trt(2-Cl)-Resin

The resin was then soaked in dimethylformamide (DMF) and controlled to pH 8 by adding diisopropylethylamine (DIEA). To the pH 8 resin, N3-PEG4-NHS (Cas No. 944251-24-5, Catalog No. J64834, manufactured by Alfa Aesar) dissolved in DMF was added and reacted to introduce an azide group (N₃-) into the N-terminus α-amino group, and the following resin was obtained.
N₃-PEG4-Gly-Thr(tBu)-Pro-Cys(Trt)-Thr(tBu)-Tyr(tBu)-Lys(Boc)-Tyr(tBu)-Nle-Leu-Ala-Glu(OtBu)-Nle-Cys(Trt)-Trt(2-Cl)-Resin

Subsequently, trifluoroacetic acid (TFA)/ultrapure water (water)/thioanisole/1,2-ethanedithiol (EDT)/triisopropylsilyl chloride (Tips) (83/5/5/5/2) was added for deprotection of the side chain of each amino acid residue and cleavage of the peptide from the resin. After the addition, the reaction was carried out at room temperature for two hours. After the reaction, the resin was filtered, and the filtrate was added to cold ether for precipitation. The precipitate was dissolved in a small amount of ultrapure water and lyophilized to obtain the following.
N₃-PEG4-Gly-Thr-Pro-Cys-Thr-Tyr-Lys-Tyr-Nle-Leu-Ala-Glu-Nle-Cys-OH (SEQ ID NO: 56)

The lyophilized product was dissolved in DMSO, and a 0.1 M aqueous NH₄CO₃ solution was added in small portions, followed by oxidation reaction with stirring at room temperature for one to two days, to form an S-S bond between the side chains of the Cys residues. The resulting product was fractionated and purified by RP-HPLC, and lyophilized to obtain the following cyclic peptide.

N3-PEG4-Gly-Thr-Pro-c(Cys-Thr-Tyr-Lys-Tyr-Nle-Leu-Ala-Glu-Nle-Cys)-OH (SEQ ID NO: 54)

The purified cyclic peptide and DBCO-PEG5-DBCO (Cas No. 2363130-04-3, Catalog No. BP-22450, manufactured by Broad Pharm) were each dissolved in dimethylsulfoxide (DMSO), mixed, and then 50 mM phosphate buffer was added. After the addition, the mixture was reacted at room temperature for two hours. The product was fractionated and purified by RP-HPLC, and lyophilized to obtain DBCO-KS-487 (theoretical molecular weight: 2701.3, measured molecular weight: 2702.0, purity: 95.0%). Here, the cyclic peptide portion excluding the modification at the N-terminus (DBCO-PEG5-Taz-PEG4) is referred to as KS-487.

DBCO-PEG5-Taz-PEG4-Gly-Thr-Pro-c(Cys-Thr-Tyr-Lys-Tyr-Nle-Leu-Ala-Glu-Nle-Cys)-OH (SEQ ID NO: 55)

### <Confirmation of Reactivity of DBCO>

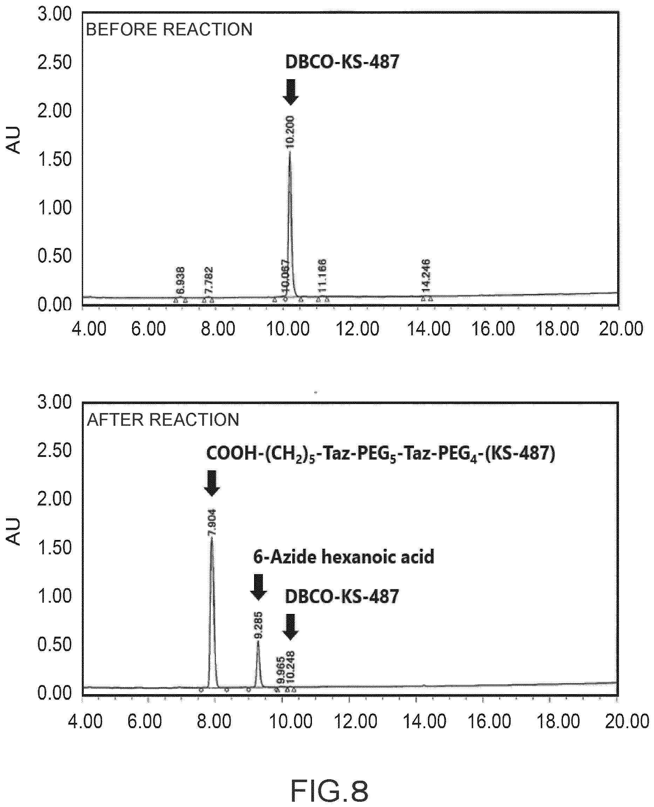

After mixing appropriate amounts of DBCO-KS-487 and 6-azido-hexanoic acid in DMSO/PBS (50/50) (pH 7.5) and reacting the mixture at room temperature, the shift of the peak position of DBCO-KS-487 was evaluated by RP-HPLC. FIG. 8 shows the results. The graph before the reaction in FIG. 8 shows the peak (10.200 min) derived from DBCO-KS-487, but the peak (10.248 min) derived from DBCO-KS-487 has almost disappeared in the graph after the reaction in FIG. 8. As shown in the graph after the reaction in FIG. 8, new peaks (7.904 min and 9.285 min) were generated. The results (before and after reaction) shown in FIG. 8 indicate that the DBCO group added to the cyclic peptide retains the activity for click reaction.

### INDUSTRIAL APPLICABILITY

The peptide of the present invention has LRP1 binding activity, and when bound to LRP1, the peptide can pass through a barrier tissue separating peripheral tissues and brain tissues, for example, the blood-brain barrier (BBB), by RMT via LRP1. Thus, the peptide of the present invention is considered to be useful for delivering any molecule to the brain tissues when combined with the molecule.

### SEQUENCE LISTING

62-11PCT_PW23008ICH-PCT.xml

## Claims

1. A linear or cyclic peptide having an amino acid sequence represented by the following formula (1) or a pharmaceutically acceptable salt thereof:
X^{N}-X⁵- X⁶-X⁷-X⁸-X⁹-X¹⁰-X¹¹-X¹²-X¹³-X¹⁴-X¹⁵ (1)
wherein X^{N} is any of one to four amino acid residues,
X⁵ and X¹⁵ each independently represent serine, threonine, cysteine, D-cysteine, or proline,
X⁶ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline,
X⁷ represents histidine, tyrosine, O-methyl-tyrosine, phenylalanine, 4-amino-phenylalanine, 4-fluoro-phenylalanine, or 4-chloro-phenylalanine,
X⁸ represents ornithine, lysine, homolysine, arginine, or homoarginine,
X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine,
X¹⁰ represents methionine, leucine, norleucine, isoleucine, valine, lysine, or algin,
X¹¹ and X¹⁴ each independently represent methionine, leucine, norleucine, isoleucine, valine, 2-aminoheptanoic acid, or 2-aminooctanoic acid, X¹² represents alanine, D-alanine, or 2-aminoisobutyric acid,
X¹³ represents glycine, alanine, asparagine, aspartic acid, glutamine, or glutamic acid, an N-terminal amino group and a C-terminal carboxy group is optionally modified or deleted, and
X⁵ and X¹⁵ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (1).

2. A linear or cyclic peptide having an amino acid sequence represented by the following formula (3) or a pharmaceutically acceptable salt thereof:
X^{N}-X⁵- X⁶-X⁷-X⁸-X⁹-X¹⁰-X¹¹-X¹²-X¹³-X¹⁴-Pro-X¹⁶ (3)
wherein X^{N} is any of one to four amino acid residues,
X⁵ and X¹⁶ each independently represent serine, threonine, cysteine, D-cysteine, or proline,
X⁶ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline,
X⁷ represents histidine, tyrosine, O-methyl-tyrosine, phenylalanine, 4-amino-phenylalanine, 4-fluoro-phenylalanine, or 4-chloro-phenylalanine,
X⁸ represents ornithine, lysine, homolysine, arginine, or homoarginine,
X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine,
X¹⁰ represents methionine, leucine, norleucine, isoleucine, valine, lysine, or algin,
X¹¹ and X¹⁴ each independently represent methionine, leucine, norleucine, isoleucine, valine, 2-aminoheptanoic acid, or 2-aminooctanoic acid,
X¹² represents alanine, D-alanine, or 2-aminoisobutyric acid,
X¹³ represents glycine, alanine, asparagine, aspartic acid, glutamine, or glutamic acid, an N-terminal amino group and a C-terminal carboxy group is optionally modified or deleted, and
X⁵ and X¹⁶ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (3).

3. A linear or cyclic peptide having an amino acid sequence represented by the following formula (3) or a pharmaceutically acceptable salt thereof:
X^{N}-X⁵- X⁶-X⁷-X⁸-X⁹-X¹⁰-X¹¹-X¹²-X¹³-X¹⁴-Pro-X¹⁶ (3)
wherein X^{N} is any of one to four amino acid residues,
X⁶ and X¹⁶ each independently represent serine, threonine, cysteine, D-cysteine, or proline,
X⁵ represents serine, homoserine, threonine, allothreonine, 2,3-diaminopropionic acid, 2,4-diaminobutanoic acid, ornithine, lysine, arginine, proline, cis-4-hydroxy-proline, or trans-4-hydroxy-proline,
X⁷ represents histidine, tyrosine, O-methyl-tyrosine, phenylalanine, 4-amino-phenylalanine, 4-fluoro-phenylalanine, or 4-chloro-phenylalanine,
X⁸ represents ornithine, lysine, homolysine, arginine, or homoarginine,
X⁹ represents methionine, norleucine, lysine, arginine, tyrosine, O-methyl-tyrosine, phenylalanine, 4-amino-phenylalanine, 4-fluoro-phenylalanine, or 4-chloro-phenylalanine,
X¹⁰ represents methionine, leucine, norleucine, isoleucine, valine, lysine, or algin,
X¹¹ and X¹⁴ each independently represent methionine, leucine, norleucine, isoleucine, valine, 2-aminoheptanoic acid, or 2-aminooctanoic acid,
X¹² represents alanine, D-alanine, or 2-aminoisobutyric acid,
X¹³ represents glycine, alanine, asparagine, aspartic acid, glutamine, or glutamic acid, an N-terminal amino group and a C-terminal carboxy group is optionally modified or deleted, and
X⁶ and X¹⁶ are cysteine or D-cysteine when involved in cyclization and form a covalent bond via a linker such as a disulfide bond, a methylene group, an acetyl methylene group, an ethylene group, or a propylene group between a side chain and an SH group, providing one cyclic structure in a molecule of the peptide of the formula (3).

4. The linear or cyclic peptide or pharmaceutically acceptable salt thereof, of any one of claims 1 to 3, wherein
X^{N} is Lys-Gly-Thr-Pro or Gly-Thr-Pro.

5. The linear or cyclic peptide or pharmaceutically acceptable salt thereof, of any one of claims 1 to 3, wherein
the amino acid sequence has one or several amino acids deleted, added, and/or substituted and has LRP1 binding activity.

6. A derivative and/or modified form of the peptide of any one of claims 1 to 3.

7. The derivative or modified form of the peptide of claim 6, wherein
the derivative or the modified form has an azide group, an alkyne group, a cyclooctyne group, or a tetrazine group bound to the N-terminus, the C-terminus, or an amino acid side chain directly or via a linker.

8. A pharmaceutical, a diagnostic agent, and/or a reagent comprising:
the peptide of any one of claims 1 to 3 or a derivative or modified form thereof.

9. A peptide-drug conjugate comprising:
the derivative or modified form of the peptide of claim 6 and a drug linked together directly or via a linker.

10. The peptide-drug conjugate of claim 9, wherein
the drug is an antibody, a nucleic acid, or a bioactive peptide.

11. A polynucleotide encoding an amino acid sequence consisting of only natural amino acids in the amino acid sequence of any one of claims 1 to 3, or a polynucleotide encoding a peptide having a base sequence with a sequence identity of 70% or more to the polynucleotide and having LRP1 binding activity.

12. An expression vector including the polynucleotide of claim 11.

13. A cell, yeast, bacterium, virus, liposome, or nanoparticle that presents the amino acid sequence of any one of claims 1 to 3.

14. A linear and cyclic peptide consisting of an amino acid sequence represented by the following formula (6) or a pharmaceutically acceptable salt thereof:
X¹-X²-X³-X⁴-X^{L}-X⁷-X⁸-X⁹-X¹⁰-X¹¹-X¹²-X¹³-X¹⁴-X^{M} (6)
wherein X^{L} is X⁵-X⁶,
X^{M} is X¹⁵-X¹⁶,
X^{L} and X^{M} each independently represent an amino acid residue having an amino group, a carboxy group, a thiol group, an allyl group, an alkynyl group, an azide group, or a halogen atom, or a derivative thereof when involved in cyclization in a peptide molecule,
X⁵, X⁶, and X¹⁵ each independently represent any amino acid residue or a derivative thereof and X¹⁶ represents any amino acid residue, a derivative thereof, or a deletion when not involved in cyclization in the peptide molecule,
X⁷ represents an amino acid residue having a hydrocarbon group that optionally has a substituent, an amino acid residue having an aromatic carbocyclic group that optionally has a substituent, an amino acid residue having an aromatic heterocyclic group that optionally has a substituent, or a derivative thereof,
X⁸ represents a positively charged amino acid residue or a derivative thereof,
X¹¹, X¹² and X¹⁴ each independently represent an amino acid residue having a hydrocarbon group that optionally has a substituent or a derivative thereof,
X¹³ represents an amino acid residue having a hydrocarbon group that optionally has a substituent, a negatively charged amino acid residue, or a derivative thereof,
X⁹ and X¹⁰ each independently represent any amino acid residue or a derivative thereof, X¹, X², X³, and X⁴ each independently represent any amino acid residue, a derivative thereof, or a deletion, a covalent bond formed between X^{L} and X^{M} directly or indirectly via a linker optionally provides one cyclic structure in a molecule of the peptide of the formula (6), the covalent bond between X^{L} and X^{M} being any of a main chain to main chain bond, a main chain to side chain bond, a side chain to main chain bond, or a side chain to side chain bond, and an N-terminal amino group and a C-terminal carboxy group is optionally modified or deleted.

15. The linear and cyclic peptide or pharmaceutically acceptable salt thereof, of claim 14, wherein
a bond between Cα-carbon atoms of X⁵ and X¹⁵, a bond between Cα-carbon atoms of X⁶ and X¹⁵, a bond between Cα-carbon atoms of X⁵ and X¹⁶, and a bond between Cα-carbon atoms of X⁶ and X¹⁶ are each independently represented by the following formula (7):
Cα^{N}-(CH₂)_{A}-Z-(CH₂)_{B}-Cα^{M} (7)
wherein N is 4 or 5, M is 14 or 15, A and B each independently represent any integer of 0 to 2, the sum of A and B is any integer of 0 to 4, and Z is S-S, CH₂-CH₂, S-CH₂, CH₂-S, O-CH₂, CH₂-O, CH=CH, NH-C(=O), N(CH₃)-C(=O), NH-C(=S), N(CH₃)-C(=S), O-C(=O), C(=O)-NH, C(=O)-N(CH₃), C(=S)-NH, C(=S)-N(CH₃), C(=O)-O, CH₂-CH₂-CH₂, S-CH₂-CH₂, CH₂-CH₂-S, CH₂-S-CH₂, O-CH₂-CH₂, CH₂-CH₂-O, CH₂-O-CH₂, S-CH₂-S, S-C(=CH₂)-S, S-(C(=O)-CH₃)-S, S-(CH₂)₂-S, S-(CH₂)₃-S, S-CH₂-C(=O)-CH₂-S, S-CH₂-C(=CH₂)-CH₂-S, S-(CH₂)₄-S, S-CH₂-CH=CH-CH₂-S, S-CH₂-C₆H₄-CH₂-S (a methylene group is optionally bound to a phenylene ring at any of ortho, meta or para), S-CH₂-C(=O)-CH₂-S, or S-CH₂-C(=CH₂)-CH₂-S.

16. The linear and cyclic peptide or pharmaceutically acceptable salt thereof, of claim 14 or 15, wherein
X⁸ is 2,3-diaminopropionic acid, 2,4-diaminobutanoic acid, ornithine, lysine, homolysine, arginine, homoarginine, D-amino acid thereof, N-methylated amino acid thereof, α-methylated amino acid thereof, or a derivative thereof.

17. The linear and cyclic peptide or pharmaceutically acceptable salt thereof, of claim 14 or 15, wherein
X¹² is alanine, D-alanine, 2-aminoisobutyric acid, 2-aminobutanoic acid, D-2-aminobutanoic acid, isovaline, D-isovaline, valine, D-valine, isoleucine, D-isoleucine, leucine, D-leucine, N-methylated amino acid thereof, α-methylated amino acid thereof, or a derivative thereof.

18. The linear and cyclic peptide or pharmaceutically acceptable salt thereof, of claim 14 or 15, wherein
X¹¹ and X¹⁴ are each independently methionine, an amino acid residue represented by the formula (8): 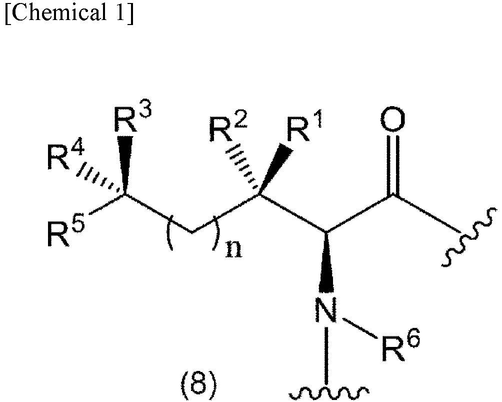 wherein a wavy line represents a point of attachment to a carbonyl group or a nitrogen atom forming an amide bond of a main chain, R¹, R², and R⁶ each independently represent a hydrogen atom or a methyl group, R³, R⁴, and R⁵ each independently represent a hydrogen atom, a methyl group, or a halogen atom (F, Cl, Br, I), and n represents any integer of 0 to 10;
the formula (9): 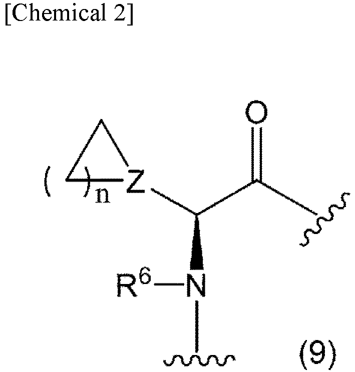 wherein Z represents C or N, R⁶ represents a hydrogen atom or a methyl group, and n represents any integer of 1 to 6;
the formula (10): 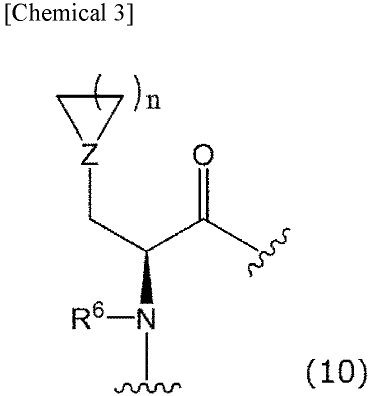 wherein a wavy line represents a site of bonding to a carbonyl group or a nitrogen atom forming an amide bond of a main chain, Z represents C or N, R⁶ represents a hydrogen atom or a methyl group, and n represents any integer of 1 to 6, or a derivative thereof.

19. The linear and cyclic peptide or pharmaceutically acceptable salt thereof, of claim 14 or 15, wherein
X⁷ is represented by the formula (8): 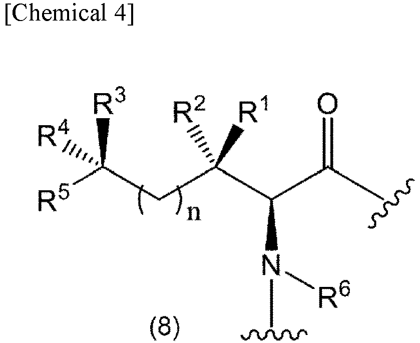 wherein a wavy line represents a point of attachment to a carbonyl group or a nitrogen atom forming an amide bond of a main chain, R¹, R², and R⁶ each independently represent a hydrogen atom or a methyl group, R³, R⁴, and R⁵ each independently represent a hydrogen atom, a methyl group, or a halogen atom (F, Cl, Br, I), and n represents any integer of 0 to 10; 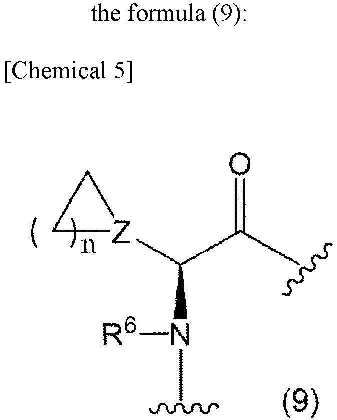 wherein Z represents C or N, R⁶ represents a hydrogen atom or a methyl group, and n represents any integer of 1 to 6;
the formula (10): 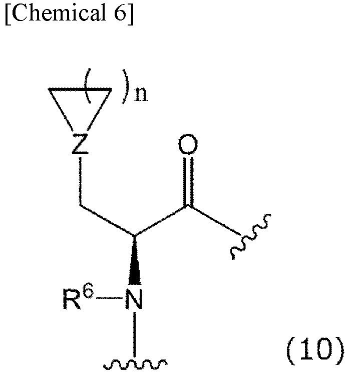 wherein a wavy line represents a site of bonding to a carbonyl group or a nitrogen atom forming an amide bond of a main chain, Z represents C or N, R⁶ represents a hydrogen atom or a methyl group, and n represents any integer of 1 to 6;
the formula (11): 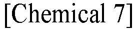 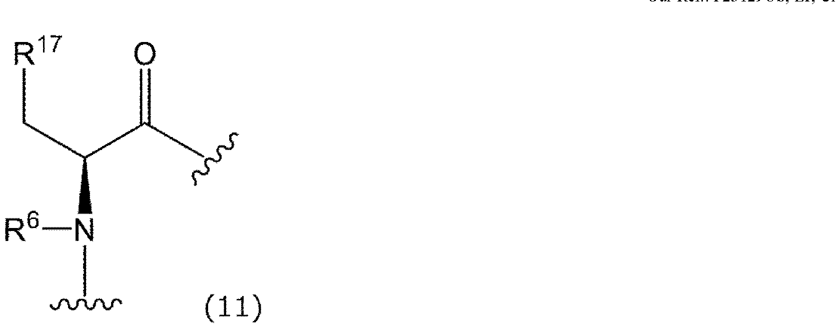 wherein R¹² represents a 2-thienyl group, a 3-thienyl group, a 2-pyridyl group, a 3-pyridyl group, a 4-pyridyl group, a 2-thiazolyl group, a 4-thiazolyl group, a 2-oxazolyl group, a 4-oxazolyl group, a 1-pyrazolyl group, a 1H-imidazol-4-yl group, a 1-imidazolyl group, a 1,2,3-triazol-1-yl group, or a 1,2,4-triazol-1-yl group, and R⁶ represents a hydrogen atom or a methyl group;
the formula (12): 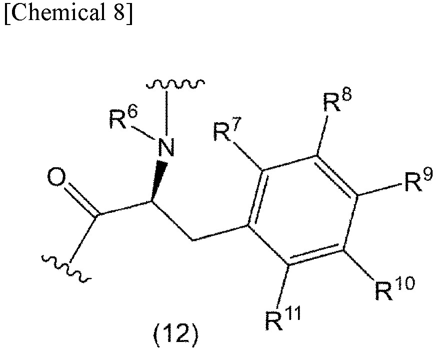 wherein a wavy line represents a point of attachment to a carbonyl group or a nitrogen atom forming an amide bond of a main chain, R⁷, R⁸, R⁹, R¹⁰, and R¹¹ each independently represent a hydrogen atom, a hydroxy group, a methoxy group, a tert-butyl group, a halogenated methyl group, or a halogen atom (F, Cl, Br, I), and R⁶ represents a hydrogen atom or a methyl group; or
the formula (13): 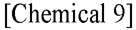 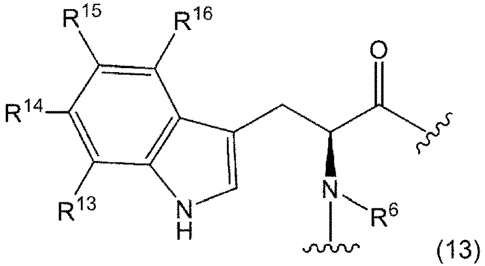 wherein R¹³, R¹⁴, R¹⁵, and R¹⁶ each independently represent a hydrogen atom, a hydroxy group, a methoxy group, a methyl group, a tert-butyl group, a halogenated methyl group, or a halogen atom (F, Cl, Br, I), and R⁶ represents a hydrogen atom or a methyl group, or a derivative thereof.

20. The linear and cyclic peptide or pharmaceutically acceptable salt thereof, of claim 14 or 15, wherein
X¹³ is glycine, alanine, aspartic acid, glutamic acid, asparagine, glutamine, D-alanine, D-aspartic acid, D-glutamic acid, D-asparagine, D-glutamine, N-methylated amino acid thereof, α-methylated amino acid thereof, or a derivative thereof.

21. The linear and cyclic peptide or pharmaceutically acceptable salt thereof, of claim 14 or 15, wherein
X^{L} and X^{M} which are not involved in cyclization in a peptide molecule are each independently alanine, isovaline, norvaline, serine, homoserine, threonine, allothreonine, O-methylated serine, O-methylated homoserine, O-methylated threonine, O-methylated allothreonine, 2,3-diaminopropionic acid, 2,4-diaminobutanoic acid, ornithine, lysine, arginine, proline, cis-4-hydroxy-proline, trans-4-hydroxy-proline, azetidine-2-carboxylic acid, pipecolic acid, D-serine, D-homoserine, D-threonine, D-allothreonine, D-O-methylated serine, D-O-methylated homoserine, D-O-methylated threonine, D-O-methylated allothreonine, D-2,3-diaminopropionic acid, D-2,4-diaminobutanoic acid, D-ornithine, D-lysine, D-arginine, D-proline, D-cis-4-hydroxy-proline, D-trans-4-hydroxy-proline, D-azetidine-2-carboxylic acid, D-pipecolic acid, N-methylated amino acid thereof, α-methylated amino acid thereof, or a derivative thereof.
